(19) Europäisches Patentamt
European Patent Office
Office européen des brevets

(11)  EP 4 077 508 B1

(12)  EUROPEAN PATENT SPECIFICATION

(45) Date of publication and mention
of the grant of the patent:
**12.11.2025  Bulletin 2025/46**

(21) Application number: **20838044.4**

(22) Date of filing: **18.12.2020**

(51) International Patent Classification (IPC):
*C08J 9/08* (2006.01)     *C08J 9/12* (2006.01)
*C08J 11/10* (2006.01)

(52) Cooperative Patent Classification (CPC):
**C08J 11/105; C08J 9/08; C08J 9/122;**
C08J 2201/03; C08J 2203/08; C08J 2300/16;
C08J 2300/30; C08J 2367/02; C08J 2367/04;
Y02W 30/62

(86) International application number:
**PCT/EP2020/087209**

(87) International publication number:
**WO 2021/123299 (24.06.2021 Gazette 2021/25)**

(54) **PROCESS FOR DEGRADING PLASTIC PRODUCTS**

VERFAHREN ZUM ABBAU VON KUNSTSTOFFPRODUKTEN

PROCÉDÉ POUR DÉGRADER DES PRODUITS EN PLASTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2019  EP 19218099**

(43) Date of publication of application:
**26.10.2022  Bulletin 2022/43**

(73) Proprietor: **Carbios
63100 Clermont-Ferrand (FR)**

(72) Inventors:
• **CHATEAU, Michel
63100 Clermont-Ferrand (FR)**

• **ALOUI DALIBEY, Madiha
63100 Clermont-Ferrand (FR)**
• **The other inventor has waived his right to be
thus mentioned.**

(74) Representative: **Cabinet Becker et Associés
25, rue Louis le Grand
75002 Paris (FR)**

(56) References cited:
**EP-A1- 1 528 079     CN-A- 102 532 647
US-A- 5 212 223     US-B1- 6 255 451**

Remarks:
The complete document including Reference
Table(s) and the Sequence Listing(s) can be
downloaded from the EPO website

**Description**

The present invention relates to a process for degrading plastic products. The process of the invention particularly comprises a step of foaming a plastic product prior depolymerizing at least one polymer of said plastic product. The process of the invention is particularly useful for degrading a plastic product comprising polyester and/or polyamide, preferably polyethylene terephthalate and/or polylactic acid. The invention also relates to a process for producing monomers and/or oligomers from a at least partially foamed plastic product.

BACKGROUND

Plastics are inexpensive and durable materials, which can be used to manufacture a variety of products that find uses in a wide range of applications (food packaging, textiles, etc.). As a consequence, the production of plastics has increased dramatically over the last decades. Moreover, most of them are used for single-use disposable applications, such as packaging, agricultural films, disposable consumer items or for short-lived products that are discarded within a year of manufacture. Because of the durability of the polymers involved, substantial quantities of plastics are piling up in landfill sites and in natural habitats worldwide, generating increasing environmental problems. For instance, in recent years, polyethylene terephthalate (PET), an aromatic polyester produced from terephthalic acid and ethylene glycol, has been widely used in the manufacture of several products for human consumption, such as food and beverage packaging (e.g.: bottles, convenience-sized soft drinks, pouches for alimentary items) or textiles, fabrics, rugs, carpets, etc.

Different solutions, from plastic degradation to plastic recycling, have been studied to reduce environmental and economic impacts correlated to the accumulation of plastic waste, including recycling technologies and energy production from such plastics. Mechanical recycling technology remains the most-used technology, but it faces several drawbacks. Indeed, it requires an extensive and costly sorting and it leads to downgrading applications, due to loss of molecular mass during the process and uncontrolled presence of additives in the recycled products. The actual recycling technologies are also expensive, so that the recycled plastic products are generally non-competitive compared to virgin plastic.

Recently, innovative processes of enzymatic recycling of plastic products have been developed and described (e.g. WO 2014/079844, WO 2015/097104, WO 2015/173265 et WO 2017/198786). Contrary to traditional recycling technologies, such enzymatic depolymerization processes allow to recover the chemical constituents of the polymer (i.e. monomers and/or oligomers). The resulting monomers/oligomers may be recovered and used to re-manufacture plastic items, so that such processes lead to an infinite recycling of plastics. These processes are particularly useful for recovering terephthalic acid and ethylene glycol from plastic products comprising PET.

However, there is always a need for processes with improved rate of degradation. One could also refer to other prior art documents. EP1528079A1 relates to a process for the production of degradable foamed lactic acid (co)polymer-based products, wherein said (co)polymers are foamed by extrusion in the presence of a foaming agent, characterised in that said foaming agent is selected from nitrogen, carbon dioxide and mixtures thereof. The lactic acid (co)polymer-based products degrade in a substantially complete manner, into CO2 and water, within a time of few weeks to about a year. US6255451B1 relates to the degradation by enzymes of various plastic products, such as foams, made of biodegradable polymers including polyester amides, and polyester urethanes. US5212223A relates to a method of making a composite foamed product, which simulates lumber, by compounding a polyolefin (e.g. HDPE) with an alkali metal bicarbonate salt and a saturated fatty acid foaming agent system in an extruder and extruding the melt to produce the end product. CN102532647A relates to a light, biological composite degradation polyethylene foam comprising LDPE, a photodegradation masterbatch, a foaming agent, a crosslinking agent and a compatibilizer. However, none of the above documents disclose the step of foaming a plastic product at a temperature at which the plastic product is in a partially or totally molten state, prior to a depolymerization step.

SUMMARY OF THE INVENTION

By working on improvements of processes for degrading plastic products, the inventors have shown that the degrading step can be improved by increasing the contact area between the plastic product and the degrading agent. The inventors have thus developed a process wherein the surface area of the plastic is increased before submitting said plastic product to the degrading step. More particularly, the inventors propose to submit the plastic product to a foaming step before the step of depolymerization. Advantageously, the foaming step allows to increase the porosity of the plastic product and thereby increases the surface area of the plastic product that can be contacted with a degrading agent and favors subsequent depolymerization of polymer(s) that composed said plastic product. The methods of the invention are particularly useful for degrading plastic products comprising polyethylene terephthalate.

In this regard, it is an object of the invention to provide a process for degrading a plastic product comprising at least one polymer comprising the steps of foaming at least partially the plastic product; and depolymerizing at least one target polymer of the at least partially foamed plastic product, wherein the step of foaming is performed at a temperature at which the plastic product is in a partially or totally molten state.

Preferably, the foaming step is performed at a temperature above the crystallization temperature (Tc) of the target polymer, preferably at or above the melting temperature (Tm) of said polymer and is implemented with a physical foaming agent and/or a chemical foaming agent.

It is also an object of the invention to provide a process further comprising a step of cooling the at least partially foamed plastic product, less than 30 seconds after the foaming step, by submitting the plastic product to a temperature below the crystallization temperature (Tc) of said polymer, preferably below the glass transition temperature (Tg) of said polymer. Advantageously, the process of the invention is performed, at least partially, in an extruder.

In an embodiment, the depolymerizing step comprises contacting the plastic product with a depolymerizing agent, selected from chemical and/or biological depolymerizing agent.

It is also an object of the invention to provide a process for degrading a plastic product comprising PET, comprising the steps of:

a. Foaming at least partially said plastic product with a foaming agent, preferably selected from chemical foaming agent, wherein the foaming step is performed at a temperature above 170°C, preferably above 185°C, more preferably above 200°C, even more preferably above 220°C, 240°C, 245°C, 250°C, 255°C, 260°C, 265°C.
b. Cooling said at least partially foamed plastic product at a temperature below 100°C, preferably below 90°C, less than 30 seconds after the foaming phase
c. Depolymerizing PET of the plastic product by contacting the plastic product to depolymerase, particularly an esterase, preferably a cutinase or a lipase, more preferably a cutinase.

According to an embodiment of the invention, the plastic product is contacted with the depolymerase before the depolymerization step (e.g. during the cooling step) and the depolymerization step comprises contacting the plastic product in a liquid deprived of depolymerase.

According to another embodiment, the depolymerization step comprises submitting the plastic product to composting conditions.

It is also an object of the invention, to provide a method of producing monomers and/or oligomers and/or degradation products from a plastic product comprising at least one polymer, preferably PET, comprising submitting successively the plastic product to a foaming step, and to a depolymerizing step, preferably comprising exposing the plastic product to a depolymerase, preferably a cutinase.

It is a further object of the invention to provide a process for degrading a at least partially foamed plastic product comprising at least one polymer, wherein the at least partially foamed plastic product is contacted with a depolymerizing agent able to degrade at least one target polymer, and optionally wherein said polymer has been submitted to an amorphizing step and the at least partially foamed plastic product is contacted with a depolymerase to degrade said polymer.

DETAILED DESCRIPTION OF THE INVENTION

*Definitions*

The present disclosure will be best understood by reference to the following definitions.

Within the context of the invention, the terms *"plastic article"* or *"plastic product"* are used interchangeably and refer to any item or product comprising at least one polymer, such as plastic sheet, tray, tube, rod, profile, shape, massive block, fiber, etc. Preferably, the plastic article is a manufactured product, such as rigid or flexible packaging (bottle, trays, cups, etc.), agricultural films, bags and sacks, disposable items or the like, carpet scrap, fabrics, textiles, etc. More preferably, plastic article refers to plastic or textile waste. Preferably, a plastic article comprises a mix of semi-crystalline and/or amorphous polymers. The plastic article may further contain additional substances or additives, such as plasticizers, minerals, organic fillers, dyes etc.

A *"polymer"* refers to a chemical compound or mixture of compounds whose structure is constituted of multiple repeating units (i.e. *"monomers"*) linked by covalent chemical bonds. Within the context of the invention, the term "polymer" refers to such chemical compound used in the composition of a plastic product. As an example, synthetic polymers include polymers derived from petroleum oil, such as polyolefins, aliphatic or aromatic polyesters, polyamides, polyurethanes and polyvinyl chloride. In the context of the invention, polymer refers to thermoplastic polymer, i.e. a polymer that becomes moldable above a specific temperature and solidifies upon cooling.

The term *"depolymerization",* in relation to a polymer or plastic article containing a polymer, refers to a process by which a polymer or at least one polymer of said plastic article is depolymerized and/or degraded into smaller molecules, such as monomers and/or oligomers and/or any degradation products.

According to the invention, *"oligomers"* refer to molecules containing from 2 to about 20 monomer units. As an example, oligomers retrieved from PET include methyl-2-hydroxyethyl terephthalate (MHET) and/or bis(2-hydroxyethyl) terephthalate (BHET) and/or 1-(2-Hydroxyethyl) 4-methyl terephthalate (HEMT) and/or dimethyl terephthalate (DMT). As another example, oligomers of lactic acid may be retrieved from PLA.

Within the context of the invention, the term *"polyester"* refers to a polymer that contain the ester functional group in their main chain. Ester functional group is characterized by a carbon bound to three other atoms: a single bond to a carbon, a double bond to an oxygen, and a single bond to an oxygen. The singly bound oxygen is bound to another carbon. According to the composition of their main chain, polyesters can be aliphatic, aromatic or semi-aromatic. Polyester can be homopolymer or copolymer. As an example, polyethylene terephthalate is a semi-aromatic copolymer composed of two monomers: terephthalic acid and ethylene glycol.

In the context of the invention, *"crystalline polymers"* or *"semi-crystalline polymers"* refer to partially crystalline polymers wherein crystalline regions and amorphous regions coexist. The degree of crystallinity of a semi-crystalline polymer may be estimated by different analytical methods and typically ranges from 10 to 90%. For instance, Differential Scanning Calorimetry (DSC) or X-Ray diffraction may be used for determining the degree of crystallinity of polymers. Other techniques are also suited for estimating with less reliability polymer's crystallinity, such as X-ray Scattering (XS) (including Small Angle and Wide Angle XS) and Infrared Spectroscopy. In the present disclosure, the degrees of crystallinity have been measured with DSC. More particularly, the DSC measures were conducted as follow: a small quantity of the sample (several mg) is heated at a constant heating rate, from ambient or sub-ambient temperature to a high temperature that is higher than the melting temperature (Tm) of the polyester. The heat flow data is collected and plotted against temperature. The degree of crystallinity Xc (%) is calculated as:

$$Xc(\%) \ = \frac{(\Delta Hf \ - \ \Delta Hcc)}{wt * \Delta Hf\,100\%} \, x\,100\%$$

where

- $\Delta H_f$ is the enthalpy of melting that can be determined by integrating the endothermic melting peak,
- $\Delta H_{cc}$ is the enthalpy of cold crystallization and determined by integrating the exothermic cold crystallization peak,
- $w_t$ the weight fraction of polyester in the plastic, and
- $\Delta H_{f,100\%}$ is the enthalpy of melting for a fully crystalline polymer and can be found in literature. As an example, $\Delta H_{f,100\%}$ of PET is taken from literature as 125.5 J/g (Polymer Data Handbook, Second Edition, Edited by James E. Mark, OXFORD, 2009). According to the literature, $\Delta H_{f,100\%}$ of PLA is equal to 93 J/g (Fisher E. W.,Sterzel H. J., Wegner G., Investigation of the structure of solution grown crystals of lactide copolymers by means of chemical reactions, Kolloid Zeitschrift & Zeitschrift fur Polymere , 1973, 251, p 980-990).

The error margin of the degree of crystallinity is about 10%. Accordingly, a degree of crystallinity of about 25% corresponds to a degree of crystallinity between 22,5% and 27,5%.

In the context of the invention, *"Tg", "Tc",* and *"Tm"* respectively refer to the glass transition temperature, the crystallization temperature, and the melting temperature of a polymer. Such temperatures may be estimated by different analytical methods. For instance, Differential Scanning Calorimetry (DSC) or Differential thermal analysis (DTA) may be used for determining the Tg, Tc, and Tm of polymers. In the present disclosure, the Tg, Tc, and Tm of polymers disclosed correspond to temperatures measured with DSC.

*Foaming step*

The inventors have shown that it is possible to improve the depolymerization rate of polymers contained in a plastic product, particularly polyesters and/or polyamides and/or polyolefins, by submitting the plastic product to a foaming step prior to submit the polymer(s) to a depolymerization step. The foaming step allows to increase the contact surface (i.e. contact area) between the polymer and the depolymerizing agent. In other words, by increasing the contact surface between the plastic product and the degrading agent, it is possible to increase the rate of depolymerization and/or to reduce the amount of degrading agent and/or to reduce the time required to degrade the plastic product as compared to same plastic product which has not been foamed. The invention particularly relates to plastic products comprising at least one thermoplastic polymer.

According to the invention, the foaming step is performed at a temperature at which the plastic product is in partially or totally molten state. Particularly, the foaming step is performed at a temperature above the crystallization temperature (Tc) of the target polymer of the plastic product (i.e. polymer for which a degradation or depolymerization is intended). Preferably, the plastic product is submitted to a temperature at or above the melting temperature (Tm) of the target polymer of the plastic product. Even more preferably, the plastic product is submitted to a temperature between Tm +5°C and Tm+25°C of the target polymer, preferably between Tm+10°C and Tm+ 25°C, more preferably between Tm+15°C and Tm+ 25°C, such as Tm+20°C of the target polymer. In another embodiment, the plastic product is submitted to a temperature between Tm +25°C and Tm+50°C of the target polymer. In another embodiment, the plastic product is submitted to a temperature corresponding to the Tm + 50°C of the target polymer or above.

According to an embodiment of the invention, the plastic product comprises several different polymers. Particularly, the plastic product comprises at least 51% by weight of the target polymer. In such case, the plastic product is advantageously submitted to a temperature at or above the Tc or to a temperature at or above the Tm of the target polymer. Alternatively, the plastic product is submitted to a temperature at or above the highest Tc or Tm of the polymers contained in the plastic product.

In a particular embodiment, the plastic product comprises PET, and the foaming step comprises submitting the plastic product to a temperature above 170°C, preferably at or above 230°C and more preferably to a temperature between 250°C and 300°C. Even more preferably, the plastic product comprising PET is submitted to a temperature between 260°C and 280°C. In another embodiment, the plastic product comprising PET is submitted to a temperature at or above 300°C, preferably between 300°C and 320°C.

In another particular embodiment, the plastic product comprises PLA, and the foaming step comprises submitting the plastic product to a temperature above 110°C and more preferably at or above 145°C. In a particular embodiment, the plastic product comprises PLLA, and the foaming step comprises submitting the plastic product to a temperature at or above 170°C. In another embodiment, the plastic product comprises stereocomplex PLA and the foaming step comprises submitting the plastic product to a temperature at or above 230°C.

As used herein, the *"foaming step"* refers to a step by which cells (also called bubbles) are created in the structure of the plastic product by use of foaming agents, also called blowing agents. Gas generated by said foaming agents creates bubbles within the molten or the partially molten plastic material, forming closed-cells and/or opened-cells in the plastic product. The resulting foamed plastic product exhibits a cellular structure, which has a lower density than the plastic product density before the foaming step.

Foaming agents can be classified as *"physical foaming agents"* or *"chemical foaming agents"*, depending on how the bubbles are generated. According to the invention, the foaming step is implemented by use of one or more foaming agents selected from physical foaming agents, chemical foaming agents and mixture thereof. In a particular embodiment, the foaming step is implemented by use of physical foaming agent(s). Alternatively, the foaming step is implemented by use of chemical foaming agent(s). In another embodiment, the foaming step is implemented by use of both physical foaming agent(s) and chemical foaming agent(s).

In the context of the invention, *"physical foaming agents"* refer to compounds that undergo a physical change of state during processing. Physical foaming agents include pressurized gases (such as nitrogen, carbon dioxide, methane, helium, neon, argon, xenon and hydrogen or mixed thereof) which expand when returning to atmospheric pressure during the process of foaming, and low-boiling-point liquids (such as pentane, isopentane, hexane, methylene dichloride, and dichlorotetra-fluoroethane) which expand when heated by changing from a liquid to a gaseous state and thereby produce a higher volume of vapor.

In a particular embodiment, the physical foaming agent is a gas. Preferably, the physical foaming agent is selected from the group consisting of nitrogen, carbon dioxide, argon, helium, methane, neon, argon, xenon, hydrogen or mixed thereof. More preferably, the physical foaming agent is selected from carbon dioxide and nitrogen. In another embodiment, the physical foaming agent is selected from the group consisting of saturated aliphatic hydrocarbons, such as methane, ethane, propane, butane, pentane and hexane; saturated alicyclic hydrocarbons, such as cyclopentane, cyclohexane, ethylcyclopentane, aromatic hydrocarbons, such as benzene, toluene, xylene; halogenated saturated hydrocarbons, such as methylene chloride, carbon tetrachloride; ethers, such as methylal, acethal, 1,4-dioxane and ketones, such as acetone, methyl ethyl ketone and acetyl ketone or a mix thereof. Alternatively, the physical foaming agent is selected from low-boiling-point liquids, selected from the group consisting of pentane, isopentane, hexane, methylene dichloride, and dichlorotetra-fluoroethane. Particularly, the low-boiling-point liquid has a boiling temperature below the temperature at which the plastic product is in partially or totally molten state. In an embodiment, the step of foaming can be implemented using one or several of the physical foaming agents listed above. In a particular embodiment, the polymer of the plastic article submitted to a foaming step with a physical foaming agent has an intrinsic viscosity index above 0.5, preferably above 0.6.

In a particular embodiment, the physical foaming agent is injected in the partially or totally molten plastic product. In other words, the plastic product is first heated and when it is molten the physical foaming agent is injected within the molten material.

In the context of the invention, *"chemical foaming agents"* refer to foaming agents that undergo a decomposition reaction during polymer heating at a given temperature, leading to the release of gas, such as nitrogen, carbon dioxide, carbon monoxide, nitroxide, NOx compounds, ammonia and/or vapor of water. Such chemical foaming agents can be selected from the group consisting of azides, hydrazides such as p,p'-hydroxybis-(benzenesulfonyl hydrazide), semicarbazides, such as p-toluenesulfonyl semicarbazide, p-toluenesulfonyl semicarbazide, azocompounds such as azodicarboxamide, triazoles, such as nitrotriazolone, tetrazoles, such as 5-phenyltetrazole, bicarbonates, such as zinc bicarbonate or alkali bicarbonates such as sodium bicarbonate, anhydride, peroxide, nitrocompounds, perchlorates. Alternatively, the chemical foaming agents are selected from citric acid, carbonate, bicarbonate and mixture thereof, or any commercial chemical foaming agent such as HYDROCEROL® from Clariant or Orgater® from Adeka. Preferably, the chemical foaming agents comprise a mix of citric acid and carbonate and/or a mix of citric acid and bicarbonate. Alternatively, the chemical foaming agent comprises hydrogen peroxide. In an embodiment, the step of foaming can be implemented using one or several of the chemical foaming agents listed above.

In a particular embodiment, the step of foaming comprises a step of mixing the chemical foaming agent(s) with the plastic product at ambient temperature and then submitting the mixture to a temperature at which the plastic product is in a partially or totally molten state.

In another embodiment, the chemical foaming agent is added to the at least partially molten plastic product. In other words, the plastic product is first heated and when it is molten the chemical foaming agent is mixed within the molten material.

In an embodiment, the foaming step is implemented with both chemical foaming agent(s) and physical foaming agent(s).

In an embodiment, the process of the invention comprises contacting from 0.1 to 10%, preferably from 0.1 to 5%, by weight of foaming agent(s) with 90% to 99.9%, preferably with 95% to 99.9%, by weight of plastic product based on the total weight of the mix foaming agents/plastic product. Particularly, the process of the invention comprises contacting from 0.1 to 10% by weight of chemical foaming agent with 90% to 99.9% by weight of plastic product based on the total weight of the mix foaming agents/plastic product. Preferably, the process of the invention comprises contacting from 1 to 5% by weight of chemical foaming agent with 95% to 99% by weight of plastic product. Alternatively, the process of the invention comprises contacting from 0.1 to 5%, preferably from 0.1 to 3%, more preferably from 0.1% to 1%, by weight of chemical foaming agent with 95% to 99.9%, preferably with 97% to 99.9%, more preferably with 99% to 99.9%, by weight of plastic product. In another embodiment, the process of the invention comprises contacting from 0.1 to 5% by weight of physical foaming agent with 95% to 99.9% by weight of plastic product based on the total weight of the mix foaming agents/plastic product. Preferably, the process of the invention comprises contacting from 0.1 to 3.5% by weight of physical foaming agent with 96.5% to 99.9% by weight of plastic product.

In an embodiment, the foaming step is implemented with foaming agent(s) and a processing aid, such as waxes, nucleation agents, chain extenders, foaming kickers or water, preferably water. Particularly, the foaming step is implemented with foaming agent(s) and from 0.01 to 10%, preferably from 0.01 to 1% by weight of processing aid, based on the total weight of the mix foaming agents/plastic product/processing aids Preferably, the foaming step is implemented with chemical foaming agent(s) and water, more preferably with a mix of citric acid and water.

In an embodiment, the foaming step is performed with an extruder, wherein the plastic product is submitted to a temperature at which the plastic product is in a partially or totally molten state.

The foaming agent may be introduced in the extruder before heating, during heating, and/or when the material has been heated and is already in a molten state.

In another embodiment, the foaming step is performed by batch foaming with an autoclave, wherein the plastic product is saturated with a foaming agent and then submitted to a sudden depressurization and optionally put into a hot oil bath. As an example, pressure-induced method or temperature-induced method could be used. Batch foaming is particularly adapted for a plastic product plastic comprising at least one polymer and an additional component that could be subjected to degradation in an extruder (for example composite comprising glass fiber or carbon fiber).

Alternatively, the foaming and/or cooling step may be carried out by any techniques known by a person skilled in the art.

Advantageously, the plastic product before the foaming step exhibits a porosity rate below 10%, preferably below 5%, more preferably below 3%. In an embodiment, the at least partially foamed plastic product exhibits a porosity rate between 20% and 90%, preferably between 25% and 50%. Particularly, the porosity rate is between 30% and 40%. Alternatively, the plastic product exhibits a porosity rate above 20%, preferably above 30%, more preferably above 40%. As used herein, the term "porosity rate" refers to the void fraction in the plastic product, and corresponds to the ratio volume of voids (i.e. pores) within the plastic product to total volume of the plastic product.

The porosity rate can be estimated by any method known by a person skilled in the art. Preferably, the "porosity rate" ($\varepsilon_T$) of the plastic product is estimated using the following equation:

$$\varepsilon_T = 1 - \frac{\rho_{app}^{water}}{\rho_p^{water}}$$

with :

- $\rho_{app}^{water}$ is the apparent density of the foamed plastic product measured using water pycnometry.

- $\cdot\rho_{p}^{water}$ is the true density of the plastic product based on its composition or measured on the unfoamed plastic composition. Particularly the plastic composition is under pellet form.

Water pycnometry consists in measuring the mass of a specific volume of water and the mass of the same volume comprising water and the foamed plastic product for which density has to be determined. This allows the determination of apparent density of the sample, giving access to the porosity rate of the material, as far as the density of the original (i.e. unfoamed) plastic product is known (i.e. true density). As an example, the true density of a plastic product comprising 100% PET from literature is 1380 kg.m-3, corresponding to the density of PET. The water pycnometry method is particularly adapted for calculating the density of products with irregular shapes. In case of products with a regular shape (e.g. cylinders) it is possible to directly calculate the volume of the product and thus assessing its apparent density. When the plastic product is a textile, the true density $\rho_{p}^{water}$ is based on the extruded but not foamed textile composition (pellet form).

In a particular embodiment, the at least partially foamed plastic product comprises at least 95% PET and exhibits an apparent density $\rho_{app}^{water}$ below 1000 kg.m-3, preferably below 900 kg.m-3. In an embodiment, the at least partially foamed plastic product exhibits a porosity rate between 40% and 70% and an apparent density $\rho_{app}^{water}$ below 1000 kg.m-3. In a particular embodiment, the plastic product comprises at least 95% PET, and the at least partially foamed plastic product exhibits an apparent density $\rho_{app}^{water}$ below 1000 kg.m-3 and a porosity rate above 30%. Preferably, the plastic product comprises at least 95% PET, and the at least partially foamed plastic product exhibits an apparent density $\rho_{app}^{water}$ below 900 kg.m-3 and a porosity rate above 30%, preferably above 40%.

In an embodiment, the at least partially foamed plastic product is a at least partially foamed textile comprising at least 85% of PET and exhibiting a porosity rate between 10% and 70%.

In an embodiment, the plastic product is submitted to a pretreatment step before the foaming step. The pretreatment step may comprise sorting and/or washing and/or disinfecting and/or sterilizing and/or biologically cleaning the plastic product prior to foaming. Alternatively or in addition, the pretreatment step may comprise physically transforming the plastic product into film, flakes, powders, pellets or fibers before foaming.

The process of the invention is particularly suited for plastic product comprising PET. It is therefore an object of the invention to provide a process for degrading a plastic product comprising at least PET and comprising a step of foaming at least partially said plastic product and a step of depolymerizing said PET of said at least partially foamed plastic product, wherein the step of foaming is preferably implemented with a chemical foaming agent, more preferably with citric acid, carbonate, bicarbonate and mixture thereof, more preferably with a mix of citric acid and carbonate or with a mix of citric acid and bicarbonate.

As mentioned above, the present invention is particularly suited for plastic products comprising thermoplastic polymers. The invention can also be implemented with plastic products comprising thermoset polymers by adapting the step of foaming.

*Cooling step*

In a particular embodiment, the process of the invention further comprises a step of cooling the at least partially foamed plastic product after the foaming step. Indeed, as exposed above, the foaming step is performed with a plastic product that is heated to be in a molten state. According to an embodiment, after the step of foaming, the foamed plastic product is submitted to a temperature colder than the temperature of the foamed plastic product, in order to quickly reduce the temperature of said foamed plastic product and to accelerate the solidification of said foamed plastic product. The cooling step comprises contacting the plastic product to any cooling fluid, including air and/or liquid.

In a particular embodiment, the plastic product is submitted to the cooling step, less than 30 seconds after the foaming step, more preferably less than 20 seconds, even more preferably less than 10 seconds. Particularly, the plastic product is submitted to the cooling step immediately after the end of the foaming (i.e. heating) step.

Such fast cooling after a heating phase allows to amorphize at least partially one or more polymer(s) of said plastic product. The amorphization takes place during the foaming step (i.e. the heating step) by allowing to break at least partially the crystalline structure of polymer(s) of the plastic product, and the fast cooling allows to fix said heated polymer in amorphous state. Amorphization of a polymer can thus be performed during the foaming step by submitting the plastic product to a temperature above the Tc, preferably above the Tm of said polymer and then rapidly cooling the plastic product at a temperature below the Tc and/or the Tg of said polymer.

As used herein, the terms *"amorphization"* and *"amorphizing"*, in connection with a polymer, refer to a decrease of the degree of crystallinity of a given polymer compared to its degree of crystallinity before amorphization. Preferably, amorphization allows to decrease the crystallinity of a target polymer of at least 5%, 10%, 15%, 20%, 25%, 30%, 35%, 40%, 50%, 60%, 70%, 80%, or 90% compared to before amorphization. Advantageously, the amorphization leads to polymer with at most 30% of crystallinity, preferably at most 25%, more preferably at most 20%, even more preferably at most 15% of crystallinity. Alternatively, amorphization allows to maintain the crystallinity of a polymer below 30%, preferably below 25%, more preferably below 20%, even more preferably below 15%. Amorphization may be performed by any process known by the person skilled in the art to break at least partially the crystalline structure of a polymer and particularly any process described in WO 2017/198786. Amorphization thereby increases the depolymerization ability of said polymer by biological agent.

The temperatures of foaming and cooling can be adapted by a person skilled in the art depending on the target polymer. Similarly, a person skilled in the art know when and/or how to perform degassing during the foaming step, before and/or after the introduction of the foaming agent. Generally speaking, the plastic product can be submitted to a heat treatment and optionally shear stress for a period of time sufficient to obtain amorphization of the target polymer. For instance, such period of time may be comprised between 10 seconds and several minutes, depending on the temperature and/or the plastic product. In a preferred embodiment, the foaming step comprises submitting the plastic product to both shear stress and a temperature above the Tc of the target polymer of the plastic product, preferably at or above the Tm of said polymer. The heating and submission to shear stress are preferably performed simultaneously to increase amorphization during the foaming step.

According to the invention, the cooling step comprises submitting the foamed plastic product to a temperature below the Tc of the target polymer of the plastic product, preferably below the Tg of said polymer. The submission to a temperature below the Tc of the target polymer of the plastic product is particularly adapted to PBAT for instance or to any polymer whose Tg is below 20°C. In another embodiment, the cooling is performed by submitting the heated plastic product to a temperature at least 20°C below the Tc of the target polymer, preferably less than at least 30°C, 40°C, 50°C. In an embodiment, the cooling is performed by submitting the plastic product to room temperature (i.e. 25°C +/- 5°C). In another embodiment, the cooling is performed by submitting the plastic product to a temperature of about 20°C, or about 10°C.

Generally speaking, the plastic product is subjected to the cooling temperature for a period of time sufficient to decrease the temperature at the very heart of the plastic product. For instance, such period of time may be comprised between 1 second and several minutes, depending on the initial temperature of the foamed plastic product (i.e. before the cooling step), and/or the cooling temperature and/or the nature/form of the plastic product. In an embodiment, the plastic product is under an extrudate form with a diameter below 1cm, preferably between 0.5 and 5 mm and is submitted to the cooling temperature for less than 1 minute, preferably less than 30 seconds, more preferably less than 20 seconds, even more preferably less than 10 seconds. Alternatively, the foamed plastic material going out of the extruder is shaped into tube or sheet.

As an example, the cooling may be performed by immersing the plastic product into a liquid at the cooling temperature, subsequently to the foaming step. For instance, the at least partially foamed plastic product is immersed into a liquid at room temperature, more preferably at a temperature below room temperature at the end of the foaming step. For instance, the plastic article is immersed in a cold liquid, whose temperature is below 14°C, preferably below 10°C or below 5°C. In a particular embodiment, the plastic product is immersed into cold water, such as water at or below 5°C. Alternatively, the plastic article is immersed in a liquid, whose temperature is below the Tc of the target polymer. More generally, any method suitable for rapidly reducing the temperature of the plastic product may be used (e.g. cold air).

In a preferred embodiment, the foaming step is performed in an extruder. The extruder allows to submit the plastic product both to a given temperature and to shear stress, simultaneously or sequentially. Advantageously, the foamed plastic product that comes out of the extruder is directly cooled by immersion and/or by pulverization of water. Advantageously, the extruder is selected from single-screw extruders, multi-screw extruders of either co-rotating or counterrotating design, planetary roller extruder, dispersive kneaders, reciprocating single-screw extruder (co-kneaders), mini extruder or internal mixer.

In an embodiment, an underwater pelletizer or an underwater strand pelletizer, allowing to cut plastic material directly in cold water, is fixed to the head of the extruder leading to the production of plastic pellets immediately submitted to the cooling phase. In such embodiment, the plastic product is under pellet form with a size below 1cm, preferably between 0.5 and 5 mm and is submitted to the cooling temperature for less than 1 minute, preferably less than 30 seconds, more preferably less than 20 seconds, even more preferably less than 10 seconds. Particularly, a microgranulation underwater pelletizer producing mini pellets under 1 mm is fixed to the head of the extruder.

Alternatively, the foaming step is performed in an autoclave and the foamed plastic product is cooled by contact with the ambient air or a cooling air or by immersion into a liquid at room temperature, or at a temperature below room temperature. Alternatively, the foaming and cooling steps may be carried out by any techniques known by a person skilled in the art.

It is therefore an object of the invention to provide a process for degrading a plastic product comprising at least one polymer, comprising the steps of:

a. Foaming at least partially the plastic product wherein the foaming step is performed at a temperature above the crystallization temperature (Tc) of a target polymer of the plastic product, preferably above the melting temperature (Tm) of said polymer; and
b. Cooling said at least partially foamed plastic product at a temperature below the Tc of said polymer, preferably below the glass transition temperature (Tg) of said polymer, and
c. Depolymerizing said target polymer.

Advantageously, the foaming agent is selected from chemical foaming agents. Preferably, the plastic product is submitted to the cooling step, less than 30 seconds after the foaming step., more preferably immediately after.

Advantageously, the at least partially foamed plastic product is submitted to a granulation step between the cooling step (b) and the depolymerization step (c), to obtain pellets as exposed above.

In a particular embodiment, the at least partially amorphized and foamed target polymer exhibits a crystallinity rate of at most 30%, preferably at most 25%, more preferably at most 20%. Preferably, the depolymerization step is performed using a biological depolymerizing agent.

In another embodiment, the extruder further comprises spinnerets for melt-spinning of nonwoven products or for melt-spinning of monofilaments or multifilaments, and the cooling step is preferably performed by use of cooling air.

It is therefore another object of the invention to provide a process for degrading a plastic product comprising at least one polymer, comprising the steps of:

Foaming and melt-spinning said plastic product wherein the foaming and melt-spinning steps are performed at a temperature above the Tc of said polymer, preferably above the Tm of a target polymer; and
Cooling said at least partially foamed and spun plastic product at a temperature below the Tc of the target polymer, preferably below the Tg of said polymer
c. Depolymerizing said target polymer,
wherein the foaming and melt-spinning steps are implemented using an extruder comprising spinneret.

Advantageously, the foaming agent is selected from chemical foaming agents and/or the plastic product is submitted to the cooling step, less than 30 seconds, preferably immediately after the spinning step, and/or the depolymerization is performed using an enzyme.

It is a particular object of the present invention to provide processes for degrading a plastic product as exposed above, wherein the plastic product comprises PET. Advantageously, the foaming step is performed in an extruder and the cooling step is performed by submitting the heated and partially foamed plastic product to a temperature below 100°C less than 30 seconds, after the foaming step, preferably below 90°C and preferably immediately after the foaming step. Alternatively, the cooling step is performed by submitting the heated plastic product to a temperature below 50°C. Particularly, the polymer is PET and the at least partially amorphized PET exhibits a crystallinity rate of at most 30%, preferably at most 25%, more preferably at most 20%.

Particularly, it is an object of the invention to provide a process for degrading a plastic product comprising at least PET, comprising the steps of:

Foaming at least partially the plastic product with a foaming agent, wherein the foaming step is performed at a temperature above 170°C, preferably above 185°C, more preferably above 200°C, even more preferably above 220°C, 230°C, 240°C, 245°C, 250°C, 255°C, 260°C, 265°C;
b. Cooling said at least partially foamed plastic product at a temperature below 100°C, preferably below 90°C, preferably less than 30 seconds after the foaming step; and
c. Depolymerizing said PET.

Advantageously, the foaming agent is selected from chemical foaming agents, preferably from citric acid, carbonate, bicarbonate or mixture thereof and/or the plastic product is submitted to the cooling step, less than 30 seconds after the foaming phase. Advantageously, the PET in the foamed product exhibits a crystallinity rate below 20% after the cooling phase, more preferably below 5%, and the depolymerizing agent is an esterase, preferably a cutinase or a lipase, more preferably a cutinase.

*Depolymerization step*

According to the invention, the degrading process comprises, following the foaming step and the optional cooling step, a step of depolymerization of at least one polymer of the plastic product. According to a preferred embodiment, the depolymerizing step targets at least one polymer that has been previously amorphized.

In a particular embodiment, the depolymerizing step comprises contacting the plastic product with a depolymerizing agent, i.e. a chemical and/or a biological agent.

Advantageously, the depolymerization step is performed in a liquid medium comprising the depolymerizing agent.

In another particular embodiment, the plastic product is contacted with a depolymerizing agent before the depolymerization step. For instance, the plastic product is immersed, after the foaming step, in a liquid comprising the depolymerizing agent. Particularly, the plastic product may be contacted with the depolymerizing agent during the cooling step (i.e. immersed in a cooling liquid comprising a depolymerizing agent). In an embodiment, the depolymerization step is subsequently performed by immersing the plastic product in a liquid. In a preferred embodiment such liquid is deprived of depolymerizing agent. In another embodiment, the depolymerization step is performed by submitting the plastic product to composting conditions. Particularly, the plastic product is submitted to industrial compost conditions at a temperature above 50°C, and/or to domestic compost conditions at a temperature between 15°C and 35°C. In an embodiment, the foamed plastic product is contacted with the depolymerizing agent during the cooling step and the depolymerization step is implemented later, by submitting the plastic product to stimuli able to activate the depolymerizing agent. For instance, the depolymerizing agent is a degrading enzyme and the stimuli consist in specific temperature and/or humidity rate.

In a particular embodiment, the depolymerizing agent is or comprises a biological agent. Particularly, the biological agent is a depolymerase (i.e. an enzyme). Preferably, the depolymerase is able to degrade at least one polymer of the plastic product, preferably at least a polymer that has been previously amorphized.

The depolymerase is advantageously selected from the group consisting of a cutinase, a lipase, a protease, a carboxylesterase, a p-nitrobenzylesterase, an esterase, a scl-PHA depolymerase, a mcl-PHA depolymerase, a PHB depolymerase, an amidase, aryl-acylamidase (EC 3.5.1.13), oligomer hydrolase, such as 6-aminohexanoate cyclic dimer hydrolase (EC 3.5.2.12), 6-aminohexanoate dimer hydrolase (EC 3.5.1.46), 6-aminohexanoate-oligomer hydrolase (EC 3.5.1.B17), oxidase, peroxidase, laccase (EC 1.10.3.2), oxygenase, lipoxygenase, mono-oxygenase, or lignolytic enzyme. In a particular embodiment, the plastic product is contacted with at least two different depolymerases.

In a particular embodiment, the plastic product comprises PET, and the depolymerase is an esterase. Particularly, the depolymerase is a cutinase, preferably a cutinase produced by a microorganism selected from *Thermobifida cellulosityca, Thermobifida halotolerans, Thermobifida fusca, Thermobifida alba, Bacillus subtilis, Fusarium solani pisi, Humicola insolens, Sirococcus conigenus, Pseudomonas mendocina* and *Thielavia terrestris,* or any functional variant thereof. In another embodiment, the cutinase is selected from a metagenomic library such as LC-Cutinase described in Sulaiman et al., 2012 or the esterase described in EP3517608, or any functional variant thereof including depolymerases listed in WO 2018/011284 or WO 2018/011281. In another particular embodiment, the depolymerase is a lipase preferably produced by *Ideonella sakaiensis.* In another particular embodiment, the depolymerase is a cutinase produced by *Humicola insolens,* such as the one referenced A0A075B5G4 in Uniprot or any functional variant thereof. In another embodiment, the depolymerase is selected from commercial enzymes such as Novozym 51032 or any functional variant thereof.

In a particular embodiment, the plastic product comprises PLLA, and the depolymerase is a protease, preferably produced by a microorganism selected from *Amycolatopsis sp., Amycolatopsis orientalis, Tritirachium album* (proteinase K), *Actinomadura keratinilytica, Laceyella sacchari LP175, Thermus sp.* or any commercial enzymes known for degrading PLA such as Savinase®, Esperase®, Everlase® or any functional variant thereof including depolymerases listed in WO 2016/062695, WO 2018/109183 or WO 2019/122308.

In another particular embodiment, the plastic product comprises PDLA, and the depolymerase is an esterase, preferably a cutinase or a lipase more preferably selected from CLE from *Cryptococcus sp.,* lipase PS from *Burkholderia cepacia, Paenibacillus amylolyticus* TB-13, *Candida Antarctica, Rhiromucor miehei, Saccharomonospora viridis, Cryptococcus magnus* or any functional variant thereof.

In another particular embodiment, the plastic product comprises PA and the depolymerase is selected from the group consisting of amidase, aryl-acylamidase (EC 3.5.1.13), oligomer hydrolase, such as 6-aminohexanoate cyclic dimer hydrolase (EC 3.5.2.12), 6-aminohexanoate dimer hydrolase (EC 3.5.1.46), 6-aminohexanoate-oligomer hydrolase (EC 3.5.1.B17).

In another particular embodiment, the plastic product comprises polyolefin and the depolymerase is an oxidase preferably selected from the group consisting of laccase, peroxidase, oxygenase, lipoxygenase, mono-oxygenase or lignolytic enzyme.

In another embodiment, the depolymerizing agent is a microorganism that expresses and excretes the depolymerase. Said microorganism may naturally synthesize the depolymerase, or it may be a recombinant microorganism, wherein a recombinant nucleotide sequence encoding the depolymerase has been inserted, using for example a vector. Particular embodiments of the depolymerization phase can be found in WO 2017/198786.

According to the invention, several microorganisms and/or purified enzymes and/or synthetic enzymes may be used together or sequentially to depolymerize different kinds of polymers contained in a same plastic article or in different plastic articles submitted simultaneously to the degrading process of the present invention.

The time required for depolymerization of at least one polymer of the plastic article may vary depending on the plastic article and the target polymer (i.e., nature and origin of the plastic article, its composition, shape, molecular weight, etc.), the type and amount of microorganisms/enzymes used, as well as various process parameters (i.e., temperature, pH, additional agents, etc.). One skilled in the art may easily adapt the process parameters to the plastic articles and/or depolymerases.

In a particular embodiment, the plastic product comprises PET, and the depolymerization step is implemented by contacting the plastic product with a biological depolymerization agent at a temperature comprised between 20°C and 90°C, preferably between 30°C and 80°C, more preferably between 40°C and 75°C, more preferably between 50°C to 75°C, even more preferably between 60°C to 75°C. Furthermore, the depolymerization step is preferably implemented at a pH between 5-11, preferably between 7-9, more preferably between 7-8.5, even more preferably between 7-8. Alternatively, the depolymerization step may be implemented under industrial and/or composting conditions.

In a particular embodiment, the plastic product comprises PLA, and the depolymerization step is implemented by contacting the plastic product with a biological depolymerization agent at a temperature comprised between 20°C and 90°C, preferably between 20°C and 60°C, more preferably between 30°C and 55°C, more preferably from 40°C to 50°C, even more preferably at 45°C. Furthermore, the depolymerization step is preferably implemented at a pH between 5-11, preferably between 7-10, more preferably between 8.5-9.5, even more preferably between 8-9. In another particular embodiment, the depolymerization step may be implemented at a pH between 7 and 8. Alternatively, the depolymerization step may be implemented under industrial and/or composting conditions.

In another particular embodiment, the depolymerizing agent is or comprises a chemical agent. Particularly, the chemical agent is a catalyst selected from metallic catalysts or stables and not toxic hydrosilanes (PMHS, TMDS) such as commercially available $B(C6F5)3$ and $[Ph3C+,B(C6F5)4-]$ catalysts. Particularly, the catalyst is selected from alkoxide, carbonate, acetate, hydroxide, alkaline metal oxide, alkaline earth metal, calcium oxide, calcium hydroxide, calcium carbonate, sodium carbonate, iron oxide, zinc acetate, zeolite. In some embodiments, the catalyst used in the depolymerization process of the present invention comprises at least one of germanium compounds, titanium compounds, antimony compounds, zinc compounds, cadmium compounds, manganese compounds, magnesium compounds, cobalt compounds, silicon compounds, tin compounds, lead compounds, and aluminum compounds. Particularly, the catalyst comprises at least one of germanium dioxide, cobalt acetate, titanium tetrachloride, titanium phosphate, titanium tetrabutoxide, titanium tetraisopropoxide, titanium tetra-n- propoxide, titanium tetraethoxide, titanium tetramethoxide, a tetrakis(acetylacetonato)titanium complex, a tetrakis(2,4-hexanedionato)titanium complex, a tetrakis(3,5-heptanedionato)titanium complex, a dimethoxybis(acetylacetonato)titanium complex, a diethoxybis(acetylacetonato)titanium complex, a diisopropoxybis(acetylacetonato)titanium complex, a di-n-propoxybis(acetylacetonato)titanium complex, a dibutoxybis(acetylacetonato)titanium complex, titanium dihydroxybisglycolate, titanium dihydroxybisglycolate, titanium dihydroxybislactate, titanium dihydroxybis(2- hydroxypropionate), titanium lactate, titanium octanediolate, titanium dimethoxybistriethanol aminate, titanium diethoxybistriethanol aminate, titanium dibutoxybistriethanol aminate, hexamethyl dititanate, hexaethyl dititanate, hexapropyl dititanate, hexabutyl dititanate, hexaphenyl dititanate, octamethyl trititanate, octaethyl trititanate, octapropyl trititanate, octabutyl trititanate, octaphenyl trititanate, a hexaalkoxy dititanate, zinc acetate, manganese acetate, methyl silicate, zinc chloride, lead acetate, sodium carbonate, sodium bicarbonate, acetic acid, sodium sulfate, potassium sulfate, zeolites, lithium chloride, magnesium chloride, ferric chloride, zinc oxide, magnesium oxide, calcium oxide, barium oxide, antimony trioxide, and antimony triacetate. Alternatively, the catalyst is selected from nanoparticules. The chemical agent can be selected from any catalyst known by a person of the art for having the capacity to degrade and/or depolymerize the target polymer.

Alternatively, the chemical agent is an acid or a base catalyst that is able to break polymer bonds, particularly esters bonds. Particularly, the chemical agent involved in breaking of esters bonds is a mixture of hydroxide and an alcohol that can dissolve the hydroxide. The hydroxide is selected from alkali metal hydroxide, alkaline-earth metal hydroxide, and ammonium hydroxide, preferably selected from sodium hydroxide, potassium hydroxide, calcium hydroxide, lithium hydroxide, magnesium hydroxide, ammonium hydroxide, tetra-alkyl ammonium hydroxide and the alcohol is selected from linear, branched, cyclic alcohol or a combination thereof, preferably linear C1-C4 alcohol selected from methanol, ethanol, propanol, butanol.

In a particular embodiment, the chemical agent is a mixture of a non-polar solvent able to swell the polymer (i.e., swelling agent) and an agent that can break or hydrolyze ester bonds, wherein the swelling agent is preferably a chlorinated solvent selected from dichloromethane, dichloroethane, tetrachloroethane, chloroform, tetrachloromethane and trichloroethane. In another particular embodiment, the chemical agent is an acid selected from ethylene glycol, hydrochloric acid, sulfuric acid or a Lewis acid.

In a particular embodiment, the at least partially foamed and optionally amorphized plastic product may be submitted to a cryogenic grinding, freezer milling, freezer grinding, or cryomilling before the depolymerization step. In an embodiment, the plastic product is crushed or grinded before the depolymerization step. Advantageously the plastic product is not submitted to a micronization step before the depolymerization step.

*Plastic articles*

The inventors have developed a degrading process for degrading plastic products comprising polymers, preferably comprising thermoplastic polymers such as polyesters and/or polyamides and/or polyolefins. The process of the invention may be advantageously used with plastic articles from plastic waste collection and/or post-industrial waste. More particularly, the process of the invention may be used for degrading domestic plastic wastes, including plastic bottles, plastic trays, plastic bags and plastic packaging, soft and/or hard plastics, even polluted with food residues, surfactants, etc. Alternatively, or in addition, the process of the invention may be used for degrading used plastic fibers, such as fibers providing from fabrics, textiles and/or and industrial wastes. More particularly, the process of the invention may be used with PET plastic and/or PET fiber waste, such as PET fibers providing from fabrics, textile, or tires. Interestingly, the process of the invention allows the production of monomers and/or oligomers and/or any degradation products that may be further recovered and/or reprocessed.

In a particular embodiment, the plastic product is selected from unfoamed plastic wastes, including plastic bottles, plastic bags and plastic packaging, soft and/or hard plastics, fibers, textiles, and/or from foamed plastic products with a crystallinity above 30% comprising thermoplastic polymers. Such foamed plastic products are submitted to a new foaming step during heating and to a cooling step for amorphization before depolymerization.

In a particular embodiment, the process of the invention is used for degrading a plastic product comprising at least one thermoplastic polymer, particularly one semi-crystalline thermoplastic polymer.

Advantageously, the process of the invention is used for degrading a plastic product comprising at least one polyester selected from polyethylene terephthalate (PET); polytrimethylene terephthalate (PTT); polybutylene terephthalate (PBT); polyethylene isosorbide terephthalate (PEIT); polylactic acid (PLA);polyhydroxyalkanoate (PHA); polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA), polyethylene naphthalate (PEN), polycyclohexylenedimethylene terephthalate (PCT), poly ethylene succinate (PES), poly (butylene succinate- co- terephtalate) (PBST), poly(butylene succinate/terephthalate/isophthalate)-co-(lactate) (PBSTIL) and blends/mixtures of these polymers. Particularly, the process of the invention is used for degrading a plastic product comprising at least one aromatic polyester selected from polyethylene terephthalate (PET); polytrimethylene terephthalate (PTT); polybutylene terephthalate (PBT); polyethylene isosorbide terephthalate (PEIT); polybutylene adipate terephthalate (PBAT), polyethylene furanoate (PEF), and blends/mixtures of these polymers.

In a particular embodiment, the process of the invention is used for degrading a plastic product comprising at least one polyester, and preferably at least PET or PLA.

Alternatively, the process of the invention is used for degrading a plastic product comprising at least one polyamide selected from polyamide-6 or poly($\beta$-caprolactam) or polycaproamide (PA6), polyamide-6,6 or poly(hexamethylene adipamide) (PA6,6), poly(11-aminoundecanoamide) (PA11), polydodecanolactam (PA12), poly(tetramethylene adipamide) (PA4,6), poly(pentamethylene sebacamide) (PA5,10), poly(hexamethylene azelaamide) (PA6,9), poly(hexamethylene sebacamide) (PA6,10), poly(hexamethylene dodecanoamide) (PA6,12), poly(m-xylylene adipamide) (PAMXD6), polyhexamethylene adipamide/polyhexamethyleneterephtalamide copolymer (PA66/6T), polyhexamethylene adipamide/polyhexamethyleneisophtalamide copolymer (PA66/6I) and blends/mixtures of these materials.

Alternatively, the process of the invention is used for degrading a plastic product comprising at least one polyolefin selected from polyethylene, polypropylene, polymethylpentene, polybutene-1, polyisobutylene, ethylene propylene rubber, ethylene propylene diene monomer rubber, ethylene vinyl alcohol, ethylene-carbon monoxide copolymer and copolymers and modifications thereof.

In a particular embodiment, the plastic product comprises at least two different polymers. More generally, the plastic products targeted by the process of the invention may comprise different kinds of polymers, including synthetic polymers, derived from petrochemicals such as polyamides, polyolefins or vinyl polymers, or biobased sourced such as rubber, wood or wood compounds such as lignin, cellulose or hemi-cellulose, and starch and derivatives thereof. Alternatively, the plastic product may comprise at least one polymer and an additional component such as metal compounds, mineral compounds, glass compounds, natural or synthetic fibers (such as glass fibers or carbon fibers), paper, and derivatives thereof as defined in WO 2015/173265.

Interestingly, the process of the invention allows to produce monomers and/or oligomers and/or degradation products that may be further recovered and/or reprocessed.

*Production of monomers/oligomers/degradation products*

It is also another object of the invention to provide a method of producing monomers and/or oligomers and/or degradation products from a plastic product comprising at least one polymer, comprising submitting successively the plastic product to a foaming step to foam at least partially said plastic product, optionally to a cooling step to amorphize at least partially said polymer of the plastic product, and then to a depolymerization step of said at least polymer in the plastic product.

It is also another object of the invention to provide a process of degrading a plastic product comprising at least one polymer, wherein the plastic product has been previously foamed, the polymer of said plastic product has been optionally at least partially amorphized and wherein the plastic product is contacted with a depolymerizing agent able to degrade said polymer, preferably a biological agent, more preferably a depolymerase. In a particular embodiment, the plastic product is depolymerized under composting conditions or under environmental conditions. Particularly, the plastic product is submitted to industrial compost conditions at a temperature above 50°C, and/or to domestic compost conditions at a temperature between 15°C and 35°C. In such case, the polymer of the plastic product may be degraded into water and/or carbon dioxide and/or methane by microorganisms in the compost and/or in the environment.

It is also another object of the invention to provide a process of degrading a plastic product further comprising a step of purification of the monomers and/or oligomers and/or degradation products resulting from the step of depolymerization. Monomers and/or oligomers and/or degradation products resulting from the depolymerization may be recovered, sequentially or continuously. A single type of monomers and/or oligomers or several different types of monomers and/or oligomers may be recovered, depending on the polymers and/or the starting plastic articles. The recovered monomers and/or oligomers and/or degradation products may be purified, using all suitable purifying method and conditioned in a re-polymerizable form. **In a** preferred embodiment, the repolymerizable monomers and/or oligomers may then be reused to synthesize polymers. One skilled in the art may easily adapt the process parameters to the monomers/oligomers and the polymers to synthesize.

It is a further object of the invention to provide a method for recycling a plastic product comprising at least one polymer, comprising subjecting successively said at least one plastic product to a foaming step and a depolymerization step, and recovering monomers and/or oligomers of such polymer.

It is also an object of the invention to provide a process for degrading a at least partially foamed plastic product comprising at least one polymer, wherein the at least partially foamed plastic product is produced from plastic waste and/or fiber waste and is contacted with a depolymerizing agent able to degrade said at least one polymer, preferably a biological agent, more preferably a depolymerase. Particularly, said at least partially foamed plastic product is obtained from plastic waste and/or fiber waste that has been previously submitted to a foaming step. Particularly, said plastic waste and/or fiber waste has been submitted to a foaming step using chemical foaming agents or physical foaming agent or both chemical and physical foaming agents. **In** a particular embodiment, said polymer of said at least partially foamed plastic product has been amorphized, and said at least partially foamed plastic product is contacted with a depolymerizing biological agent, preferably a depolymerase to degrade said amorphized polymer. Said foaming and amorphizing steps may be performed according to particular embodiments exposed above.

Particularly, it is an object of the invention to provide a process for recycling a plastic product selected from plastic waste and/or fiber waste and comprising at least one polymer, wherein said plastic waste and/or fiber waste has been previously foamed, said process comprising a step of depolymerizing said at least partially foamed plastic product by contacting said product with a depolymerizing agent able to degrade said at least one polymer, preferably a biological agent, more preferably a depolymerase. In an embodiment, the plastic product selected from plastic waste and/or fiber waste has been previously foamed according to particular embodiments exposed above. Particularly, said plastic product has been previously foamed by use of chemical foaming agent(s) or physical foaming agent(s) or both chemical and physical foaming agents. In a particular embodiment, said polymer of said plastic product has been previously amorphized before being in contact with the depolymerizing agent, preferably with a biological agent, more preferably with a depolymerase able to degrade said amorphized polymer. In an embodiment, the plastic product selected from plastic waste and/or fiber waste has been previously amorphized according to particular embodiments exposed above.

It is thus an object of the invention to use a foamed plastic product comprising at least one polymer and submitting such foamed plastic product to a depolymerisation step to produce monomer and/or oligomers of such polymer. Preferably, said foamed plastic product comprises plastic waste and/or fiber waste that has been previously foamed and whose polymer has been optionally previously amorphized.

All particular embodiments exposed above in connection with the process for degrading plastic product also apply to the methods of producing monomers and/or oligomers and to the methods of recycling.

*Biodegradable plastic production*

It is another object of the invention to provide a plastic product comprising at least one target polymer and incorporating at least one enzyme able to degrade said target polymer, wherein the enzyme has been incorporated in the plastic product according to the following process:

a. Foaming at least partially said plastic product with a foaming agent, preferably selected from chemical foaming agent, wherein the foaming step is performed at a temperature above the Tc, preferably above the Tm of said target polymer

b. Cooling said at least partially foamed plastic product, less than 30 seconds after the foaming step, by submitting the plastic product to a liquid comprising a depolymerizing agent (i.e., an enzyme able to degrade the target polymer), at a temperature below the Tc and/or the Tg of said target polymer.

Further aspects and advantages of the invention will be disclosed in the following examples, which should be considered as illustrative and do not limit the scope of this application. These Examples provide experimental data supporting the invention and means of performing the invention.

EXAMPLES

**Example** 1 - **Process of degrading a plastic product comprising PET including a foaming step performed with a chemical foaming agent**

A) Foaming step with chemical foaming agent (CFA) and subsequent cooling step

a. With HYDROCEROL PEX 5048 from Clariant as CFA

Washed and colored flakes from bottle waste comprising 98% of PET with a mean value of crystallinity of 34.5% were foamed using a twin-screw extruder Leistritz ZSE 18 MAXX, which comprises nine successive heating zones (Z1 - Z9) and a head (Z10) wherein the temperature may be independently controlled and regulated in each zone.
The flakes were introduced in the principal hopper (before Z1). Chemical foaming agent HYDROCEROL PEX 5048 from Clariant was introduced in Z4 using a gravimetric feeder. A total flow rate of 3 kg/h was obtained leading to an extruded composition (S1) containing 4% of CFA based on the total weight of said composition. The screw speed rate was set to 200 rpm.

b. With citric acid as CFA

Ground and washed colored flakes comprising 98% of PET with a mean value of crystallinity of 34.5% were dry blended with 1% by weight of citric acid (Orgater exp 141/183 from Adeka) in powder form, based on the total weight of said composition, leading to an extruded foamed composition (S1 BIS). The screw speed rate was set to 110 rpm, and total flow rate to 4 kg/h.
Temperature profile along the screw for preparation of sample S1 and S1 BIS are described in Table1.

Table 1 : Temperature profile of extruder used for sample named S1 and S1 BIS

| Sample | Zone | Z1 | Z2 | Z3 | Z4 | Z5 | Z6 | Z7 | Z8 | Z9 | Z10 (head) |
|---|---|---|---|---|---|---|---|---|---|---|---|
| S1 | T°C | 260°C | 260°C | 250°C | 250°C | 250°C | 250°C | 240°C | 240°C | 240°C | 240°C |
| S1 BIS | T°C | 250°C | 260°C | 280°C | 280°C | 270°C | 260°C | 260°C | 230°C | 230°C | 230°C |

The molten polymer arrived in the screw head (Z10) comprising a die plate with one hole of 3.5 mm and was immediately immersed in a 2 m long cold water bath (10°C). The resulting extrudate was granulated into 2-3 mm solid pellets (samples S1 and S1 BIS) with a crystallinity level of 0% and 1% respectively.
The porosity rate $\varepsilon_T$ of each sample was calculated using the following equation:

$$\varepsilon_T = 1 - \frac{\rho_{app}^{water}}{\rho_p^{water}}.$$

with :

- $\rho_{app}^{water}$ is apparent density measured using water pycnometry.

- $\rho_p^{water}$ is the true density measured on unfoamed polymer.

The porosity rate $\varepsilon_T$ of S1 is 33.6% and 54,6% for S1 BIS.

Water pycnometry is determined as defined in the description using 4 to 5 extrudates of 1 to 2 cm long, corresponding to 1 to 2 g of material.

A control sample "Control-1" was extruded and granulated in the same conditions as S1 without the use of foaming agent. Control-1 has a porosity rate $\varepsilon_T$ of 0. Control-1 has a crystallinity level of 15%.

A second control sample "Control-2" (fine powder form) with crystallinity level of 15% was prepared by immersing the pellets of Control-1 in liquid nitrogen and micronizing said pellets using RETSCH ZM 200 Ultra-Centrifugal Mill equipped with 500 $\mu$m grid. Only the powder with a size inferior to 500$\mu$m obtained by sieving was used for depolymerization step.

B) Depolymerization step of the foamed plastic product

The depolymerization process was carried out in 500 ml Mini-bioreactors (Global Process Concept, France) using a variant of LC-cutinase (Sulaiman et al., Appl Environ Microbiol. 2012 Mar). Such variant (LCC-ICCIG) corresponding to the enzyme of SEQ ID N°1 with the following mutations F208I + D203C + S248C + V170I + Y92G was expressed as recombinant protein in *Trichoderma reesei.*

100mg of a variant of LC-cutinase prepared in 224 ml of 100 mM potassium phosphate buffer, pH 8, was combined with 56 g of PET samples. Temperature was regulated at 60°C and a marine turbine was used to restrain constant agitation at 250 rpm. The pH was regulated to 8 with 6 N NaOH and controlled by the GX controler with the C-BIO™ software (Global Process Concept, France), and the consumption of NaOH was recorded during the process time.

The depolymerization rate of PET was determined *via* regular sampling. The samples were analyzed by Ultra High Performance Liquid Chromatography (UHPLC) for measuring the amount of terephthalic acid equivalent produced according to the method described herein.

The AT equivalent concentration was determined by chromatography (UHPLC). If necessary, the samples were diluted in 100 mM potassium phosphate buffer, pH 8. One mL of samples or diluted samples were mixed with 1 mL of methanol and 100 $\mu$L of 6 N HCl. After homogenization and filtration through a 0.45 $\mu$m syringe filter, 20 $\mu$L of sample were injected into the UHPLC, Ultimate 3000 UHPLC system (Thermo Fisher Scientific, Waltham, MA) including a pump module, a sampler automatic, a column thermostated at 25 °C and a UV detector at 240 nm. The terephthalic acid (AT) and the produced molecules (MHET and BHET) were separated using a gradient of methanol (30% to 90%) in 1 mM $H_2SO_4$ at 1 m / min through a HPLC Discovery HS C18 column (150 mm x 4.6 mm, 5 $\mu$m) equipped with a precolumn (Supelco, Bellefonte, PA). AT, MHET and BHET were measured according to standard curves prepared from commercially available AT and BHET and internally synthesized MHET. The AT equivalent is the sum of the measured TA and the TA equivalent in the measured MHET and BHET. The percentage of hydrolysis of samples S2 and Control2 was calculated based on the total amount of TA equivalent (TA +MHET + BHET) at a given time versus the total amount of TA determined in the initial sample. Results of percentage of depolymerization are shown in Table 2 below.

Table 2: PET depolymerization rate of a foamed plastic product comprising PET (S1 and S1 BIS) compared to an unfoamed extruded plastic product (Control-1), and an unfoamed, extruded and micronized plastic product (Control-2).

| Sample | Percentage of depolymerization (%) at 12h |
|---|---|
| **Control-1** | 2 |
| **Control-2** | 48 |
| **S1** | 60 |
| **S1 BIS** | 65 |

The results firstly show that a foaming step enables to increase from 30 times the percentage of depolymerization of PET compared to the unfoamed plastic product. Moreover, the results also show that the process of the invention enables to suppress the micronization step since the foamed plastic product is depolymerized faster than the unfoamed, extruded and micronized plastic composition (Control-2).

**Example 2 - Process of degrading a plastic product comprising PET including a foaming step performed with physical foaming agent**

A) Foaming step with carbon dioxide (CO2) and subsequent cooling step

Washed and colored flakes from bottle waste comprising 98% of PET were foamed with supercritical $CO_2$ using a single screw extruder. Such extruder (diameter 30 mm - SCAMEX, FRANCE) comprises six heating zones (T) wherein the temperature may be independently controlled and regulated in each zone:

- T1 and T2: zones before $CO_2$ injection,
- T3 and T4: zones after $CO_2$ injection,
- T5: mixing zone comprising a static mixer and
- T6: die which comprises a die plate with an opening that can be adjusted according to the outlet pressure with maximum opening of 3 mm.

Temperatures in T1 to T3 are fixed to 180°C, 280°C and 260°C respectively, and the temperatures in T4 to T6 are listed in Table 3 below. The screw speed rate was fixed at 40 rpm.

Pressure in the final part of the mixing zone (T5) is measured by a pressure sensor and indicated in Table 3 (P4). $CO_2$ is pressurized and injected at constant flow using a syringe pump (Isco 260D, USA) between T2 and T3. Pressure, temperature and volumetric input of $CO_2$ ($Q_{CO2}$) are measured in the pump and indicated in Table 3.

The obtained extrudates are immediately immersed in fresh water at about 15°C and then cut into pieces of 2-3 mm with a knife mill. The obtained samples were then dried at ambient conditions during 48h before analysis.

Other experimental conditions used for samples preparation and porosity and crystallinity results are shown in Table 3 below. Qp is the polymer flow rate which was determined by weighing obtained sample. $Q_{CO2}$ is the volume flow of injected $CO_2$. The mass flow rate of $CO_2$ relative to the total flow rate ($w_{CO2}$) is calculated using the density obtained by equation of state of Span and Wagner (R. Span et W. Wagner, A new equation of state for carbon dioxide covering the fluid region from the triple-point temperature to 1100 k at pressures up to 800 mpa. Journal of Physical and Chemical Reference Data, vol. 25(6), pp. 1509-1596, 1996). Tmat is the measured material temperature at the exit of the extruder.

Table 3: experimental conditions used for foamed plastic products preparation using physical foaming agent and porosity and crystallinity results

| Sample | T4 (°C) | T5 (°C) | T6 (°C) | P4 (bar) | Tmat3 | Qp (g/min) | $Q_{CO2}$ (ml/min) | $W_{CO2}$ (%) | $\rho_{app}^{water}$ (kg/m$^3$) | $\varepsilon_T$ (%) | Crystallinity (%) |
|---|---|---|---|---|---|---|---|---|---|---|---|
| S2 | 255 | 255 | 255 | 117 | 241 | 18 | 0.35 | 1.8 | 741.4 | 46 | 20 |
| S3 | 255 | 255 | 255 | 110 | 240 | 27.2 | 0.3 | 1.0 | 627.9 | 54 | 14 |
| S4 | 240 | 240 | 240 | 107 | 229 | 19.4 | 0.5 | 2.4 | 664.9 | 51 | 9 |
| S5 | 230 | 230 | 230 | 124 | 219 | 17 | 0.3 | 1.7 | 804.3 | 41 | 13 |

B) Foaming step with azote (N2) and subsequent cooling step

Extrusion-foaming step has been realized using a single screw extruder (diameter 45 mm, FAIREX) comprising a melt cooler, a static mixer and a vertical die with a nominal diameter of 2 mm. A PROMIX injection gas system was used to introduce supercritical $N_2$. A 2 m long cold water bath (9°C) was used to cool the extrudate before granulation with a rotary cutter to obtain 1,5 mm long pellets. The distance between the exit of the die and the water surface was about 5.5 cm.

Washed and colored flakes from bottle waste comprising 95% of PET (crystallinity 43%) were dried at 80°C. Set temperature and recorded material parameters are shown in Table 4 and 5 respectively.

The screw speed rate was set to 25 rpm. The total material flow rate was 6 kg/h and the injected nitrogen flow rate was 15 g/h. The obtained pellets S6 were then dried at ambient temperature for 48h before analysis.

Table 4: Set of temperatures in the extruder

| T1 (°C) | T2 (°C) | T3 (°C) | T4 (°C) | T5 (°C) | T_Melt cooler (°C) | T_Static mixer (°C) | T Die (°C) |
|---|---|---|---|---|---|---|---|
| 240 | 245 | 250 | 250 | 250 | 240 | 245 | 215 |

Table 5: Material parameters recorded at the end of screw and at the static mixer exit

| End of screw | | Static mixer exit | |
| --- | --- | --- | --- |
| Pressure (bar) | Temperature (°C) | Pressure (bar) | Temperature (°C) |
| 146 | 223 | 126 | 217 |

The $\rho_{app}^{water}$ of obtained sample S6 was evaluated to 461,7 kg/m$^3$ which gives a porosity rate of 66%. Its crystallinity level was evaluated to 14%.

C) Depolymerization step

The same secreted recombinant LCC-ICCIG enzyme as in Example 1 was used for the subsequent depolymerization of samples S2 to S6 and Control-1 (produced as in Example 1). For each sample from S2 to S6 and Control-1, 100 mg were respectively weighted and introduced in a 250 ml glass bottle containing 49 mL of 0.1 M potassium phosphate buffer (pH 8). The depolymerization was started after the addition of 1 mL of enzymatic solution at 0.1 mg/mL in 0.1 M potassium phosphate (pH 8) by incubating each sample at 60°C and 150 rpm in a Multitron pro (Infors HT, Switzerland).
The depolymerization rate of PET was determined *via* regular sampling and samples were analyzed by Ultra High Performance Liquid Chromatography (UHPLC) for measuring the amount of terephthalic acid equivalent produced according to the method described in Example 1. The percentage of hydrolysis of samples S2 to S6 and Control-1 was calculated based on the total amount of TA equivalent (TA +MHET + BHET) at a given time versus the total amount of TA determined in the initial sample. Results of percentage of depolymerization are shown in Table 6 below.

Table 6: PET depolymerization rate of a foamed plastic product comprising PET (S2 to S6) compared to an unfoamed plastic product (Control-1).

| Sample | Percentage of depolymerization (%) at 28h |
| --- | --- |
| **Control-1** | 6.0 |
| **S2** | 32.9 |
| **S3** | 46.5 |
| **S4** | 39.8 |
| **S5** | 20.5 |
| **S6** | 49.1 |

The degrading process of a plastic product comprising PET that has been previously foamed using a physical foaming agent (carbon dioxide or azote) is improved from 3 to 8 times from a plastic product which has not be submitted to foaming.

**Example 3 - Process of degrading a plastic product comprising PET including a foaming step performed with physical foaming agent and a step of cooling in a bath containing enzyme solution**

A) Foaming step with supercritical CO2 and subsequent cooling step

Washed and colored flakes from bottle waste comprising 98% of PET were foamed with supercritical CO2 according to example 2-A.
Two samples, S7 and S8, were prepared according to the indication detailed in Table 7. The extrudate was either immersed in water (S7) or in water comprising enzyme bath containing approximately 4.3 g/l of same secreted recombinant LCC-ICCIG enzyme as in Example 1 (S8). The obtained extrudates were rinsed with water, dried under ambient conditions and then cut into pieces of 2-3 mm with a knife mill.
Experimental conditions used for sample preparation and porosity and crystallinity results are shown in table 7 below:

Table 7: Experimental conditions used for foamed plastic products preparation and porosity and crystallinity results

| Sample | T4 (°C) | T5 (°C) | T6 (°C) | Qp (g/min) | $Q_{CO2}$ (ml/min) | $W_{CO2}$ (%) | Cooling bath | $\rho_{app}^{water}$ (kg/m$^3$) | $\varepsilon_T$ (%) | crystallinity (%) |
|---|---|---|---|---|---|---|---|---|---|---|
| S7 | 250 | 250 | 250 | 11,43 | 0.3 | 2.4 | water | 582.3 | 57 | 16 |
| S8 | 250 | 250 | 250 | 20 | 0.3 | 1.4 | water + enzyme | 627.9 | 42 | 16 |

B) Depolymerization step

Depolymerization was carried on S7 and S8 in glass bottles as detailed in Example 2-B, except that S8 was tested without adding enzyme in the buffer. Results of depolymerization are shown in Table 8.

Table 8: PET depolymerization rate of a foamed plastic product comprising PET submitted to cutinase during the depolymerization step (S7), compared to a foamed plastic product comprising PET submitted to cutinase during the cooling step, with no addition of cutinase during the depolymerization step (S8).

| Sample | Percentage of depolymerization (%) at 30h |
|---|---|
| S7 | 75 |
| S8 | 85 |

The degrading process of a plastic product comprising PET that has been submitted to depolymerase during the cooling step show a slightly increase in degradation compared to the one contacted with the depolymerase only during the depolymerization step.

**Example** 4 - **Process of degrading a plastic product comprising PLA including a foaming step with chemical foaming agent and step of cooling in a bath containing enzyme solution**

A) Foaming step with chemical foaming agent

Polylactic acid (PLA) 4043D (pellet form provided from NatureWorks - crystallinity 35%) was foamed using a twin-screw extruder Leistritz ZSE 18 MAXX was used. It comprises nine successive heating zones (Z1 - Z9) and a head (Z10) wherein the temperature may be independently controlled and regulated in each zone. A chemical foaming agent (CFA) HYDROCEROL BIH 40 masterbatch provided by Clariant was used.

PLA and CFA were dried in desiccator during 14h at 60°C and 45°C respectively. 95% by weight of PLA pellets and 5% of the CFA masterbatch were dry blended and added to the hopper of a gravimetric feeder to be introduced to the extruder. A total flow rate of 2 kg/h was obtained. Temperature profile all along the screw is described in Table 9. The screw speed rate was set to 100 rpm.

Table 9: Temperature profile of extruder used for sample named S9

| Zone | Z1 | Z2 | Z3 | Z4 | Z5 | Z6 | Z7 | Z8 | Z9 | Z10 (head) |
|---|---|---|---|---|---|---|---|---|---|---|
| T°C | 140°C | 140°C | 150°C | 170°C | 170°C | 170°C | 170°C | 170°C | 170°C | 165°C |

The molten polymer arrived in the screw head (Z10) comprising a die plate with one hole of 3.5 mm. The resulting extrudate was immediately immersed in a container with 1 L of commercially available enzyme solution Savinase® 16L from Novozymes (known to be able to degrade PLA) with temperature 15°C, then pulled and wound manually. 24 hours later, the sample was washed with water and dried at ambient conditions (20°C and 40% humidity) during 48 hours. The sample was then granulated with a rotary cutter into 2-3 mm solid pellets (S9) with a crystallinity of 2%. As a control, another sample (S10) was also foamed in the same manner except that the resulting extrudate was immersed in water deprived of enzyme. S9 and S10 both exhibit a crystallinity of 2%, and a porosity rate of 30% and 40% respectively.

B) Depolymerization step

100 mg of each alloy have been weighted and introduced in cellulose dialysis tubing. This latter was introduced in a glass bottle containing 50 mL of Tris 100mM buffer pH 9.5, incubated at 45°C and 150 rpm.

Degradation percentage of the alloy has been performed by UHPLC according to the protocol below. 1mL samples have been regularly taken. After filtration on 0.22 $\mu$m filter, samples were loaded on UHPLC (Ultimate 3000 UHPLC system (Thermo Fisher Scientific, Inc. Waltham, MA, USA) including a pump module, an autosampler, a column oven thermostated at 50°C, and a UV detector at 210 nm) to monitor the liberation of lactic acid and dimers of lactic acid. Lactic acid and dimers of lactic acid were separated using column Aminex HPX-87H and a mobile phase H2SO4 5 mM, at a flow rate of 0.5 mL.min-1. Injection was 20 $\mu$L of sample. Lactic acid (LA) and dimers of lactic acid (DP2) were measured according to standard curves prepared from commercial lactic acid (Sigma-Aldrich L1750-10G) and in-house synthetized dimers of lactic acid in the same conditions than samples.

The degradation percentage was calculated according to the following molar ratio of LA plus the LA contained in DP2 at a given time versus the theorical LA contained initially in the PLA.

After 24 hours, S9 show a degradation rate of 76%, whereas S10 show no significant degradation. The results indicate that some enzymes have been fixed in the cell structures of the foamed plastic material comprising PLA during the cooling phase.

## Example 5 - Process of degrading a textile product comprising PET including a foaming step with a chemical foaming agent

### A) Foaming step of a textile product comprising PET

Textile wastes (old clothes) composed of PET were sorted, shredded, cleaned to eliminate their metals and hard contaminants (such as zip or buttons) and compacted to obtain textile granules of 2-4 mm size comprising 85% by weight of PET.

The same extruder, cooling and granulation equipment as for Example 1 were used to prepare foamed pellets from said PET textile granules.

Compacted textile granules were introduced in the principal hopper (before Z1). Citric acid (Orgater exp 141/183 from Adeka) was used as CFA and was introduced in Z4 using a gravimetric feeder. The screw speed rate was set to 110 rpm. A total flow rate of 4 kg/h was obtained leading to an extruded composition (S11) containing 1% by weight of citric acid based on the total weight of said composition.

A control sample from old clothes composition "Control-3" was extruded, cooled and granulated without the use of foaming agent and then micronized after immersion in liquid nitrogen to obtain a powder with 13% of crystallinity level. The set temperature profile for extrusion is given in Table 10. The screw speed rate was set to 200 rpm and the total flow rate to 4 kg/h.

A woven fabric containing 100% PET was shredded and compacted then dry blended with 1% by weight of citric acid based and 0.5% by weight of water based on the total weight of the mixture, before extrusion foaming. The same extruder as for S11 and Control-3 sample was used. The dry blend was introduced via a gravimetric doser in the principal hopper (before Z1). A total flow rate of 2.5 kg/h was obtained leading to an extruded composition (S12) containing 1% by weight of citric acid based on the total weight of said composition. The screw speed rate was set to 150 rpm.

Temperature profile along the screw for samples S11, S12 and Control-3 are shown in Table 10 below.

Table 10: Temperature profile of extruder used for samples S11, S12 and Control-3

| Sample | Zone | Z1 | Z2 | Z3 | Z4 | Z5 | Z6 | Z7 | Z8 | Z9 | Z10 (head) |
|---|---|---|---|---|---|---|---|---|---|---|---|
| S11 | T°C | 250°C | 250°C | 260°C | 220°C | 230°C | 270°C | 270°C | 250°C | 230°C | 230°C |
| S12 | T°C | 260°C | 260°C | 250°C | 250°C | 250°C | 250°C | 240°C | 240°C | 240°C | 240°C |
| Control-3 | T°C | 265°C | 265°C | 265°C | 255°C | 255°C | 250°C | 250°C | 245°C | 245°C | 245°C |

S11 and S12 have a crystallinity level of 13% and 0% respectively and a porosity rate of 25% and 36% (with the true density measured on an extruded but unfoamed textile composition).

### B) Depolymerization step of the foamed textile product

Depolymerization step was conducted under same conditions as in example 1)-B.

The percentage of depolymerization that were obtained are summarized in Table 11 below.

Table 11: PET depolymerization rate of foamed textile products comprising PET (S11 and S12) compared to an unfoamed, extruded and micronized textile (Control-3).

| Sample | Percentage of depolymerization (%) at 44h |
|--------|-------------------------------------------|
| S11 | 89 |
| S12 | 90 |
| Control-3 | 85 |

The results show that the process of the invention allows to suppress the micronization step since the foamed textile product is depolymerized as much as the unfoamed, extruded and micronized textile composition (Control-3).

**Example 6 - Process of degrading a plastic product comprising PET including a foaming step performed with a chemical foaming agent and a depolymerizing step performed with a chemical depolymerizing agent**

A) Foamed plastic product preparation

In said example, the following materials were used

- Sample S1 BIS which corresponds to the ground, washed and colored flakes foamed with citric acid, as described in Example 1-A)b)
- Sample Control-1 which corresponds to the extruded and granulated but unfoamed flakes (see Example 1)
- Control-2 which was obtained by micronization of the flakes of Control-1 (see Example 1)
- Control-4 which corresponds to Control-1 that has been submitted to an annealing treatment in an oven at 120°C during 48 hours in order to recrystallize the pellet. Control-4 exhibits a crystallinity level of about 32.2%.
- Control-5 which was obtained by micronization of the pellets of Control-4 (using the methods described in Example 1).

B) Chemical depolymerization of the foamed plastic products

The chemical depolymerization was carried out in 15 mL glass tubes (Supelco, 27162) with screw cap. A sample of ~ 40-50 mg of PET was placed in a glass tube and a total of 800 $\mu$L of DCM and 400 $\mu$L of methanol/KOH (3M) were added. The mixture was stirred during 5 minutes by magnetic stirring at room temperature (RT ~ 25°C). The solvents were evaporated under a flow of N2 for 10 min. The PET monomers were dissolved in 14 mL of milliQ water. The solution was stirred for 5 min at RT. The amount of Mono-ethylene glycol (MEG) produced was determined by Ultra High-Performance Liquid Chromatography (UHPLC) according to the method described herein.

The MEG concentration was determined by mixing 1.5 mL of sample with 0.5 mL of $H_2SO_4$. After homogenization and filtration through a 0.45 $\mu$m syringe filter, 20 $\mu$L of sample were injected into the UHPLC, Ultimate 3000 UHPLC system (Thermo Fisher Scientific, Waltham, MA) including a pump module, a sampler automatic, a column thermostated at 55°C and a RI detector. The MEG molecules were separated by means of a HPLC Aminex HPX-87H ion exclusion column (300 mm x 7.8 mm, 9 $\mu$m) equipped with a precolumn (Supelco, Bellefonte, PA). MEG was eluted with H2S04 5mM using a flow rate of 0.8 mL min$^{-1}$._MEG was measured according to standard curves prepared from commercially available MEG. The velocity of reactions was calculated based on the total amount of MEG at a given time versus the total amount of MEG determined in the initial sample. Results of velocity of reaction are shown in Table 12 below, as well as the crystallinity level for each sample.

Table 12: PET chemical depolymerization velocity of foamed plastic product comprising PET (S1-BIS) compared to unfoamed and extruded product (Control-1), unfoamed, extruded and micronized product (Control-2), recrystallized pellets (Control-4) and recrystallized and micronized product (Control-5)._Velocity of chemical reaction is expressed in mg/min.

| Sample | Description | Crystallinity level of the sample (%) | Velocity of reaction in mg/min |
|--------|-------------|---------------------------------------|--------------------------------|
| S1-BIS (from Example 1) | Foamed plastic pellet | 1.0 | 9.6 |
| Control-1 | Unfoamed, extruded, granulated plastic pellet | 15.0 | 3.9 |

(continued)

| Sample | Description | Crystallinity level of the sample (%) | Velocity of reaction in mg/min |
|---|---|---|---|
| Control-2 | Unfoamed, extruded, granulated plastic pellet after micronization | 15.0 | 10 |
| Control-4 | Annealed plastic pellet | 32.2 | 0.3 |
| Control-5 | Annealed plastic pellet after micronization | 31.8 | 7.9 |

The results show that the foamed plastic product is depolymerized faster than the unfoamed plastic product, and faster than the annealed (recrystallized) pellet with or without micronization. Results also show that the process of the invention allows to suppress the micronization step since the foamed product is depolymerized as much as the unfoamed, extruded and micronized product.

**Claims**

1.  A process for degrading a plastic product comprising at least one polymer, said process comprising the steps of:

    a. Foaming at least partially the plastic product; and
    b. Depolymerizing at least one target polymer of the at least partially foamed plastic product,

    wherein the step of foaming is performed at a temperature at which the plastic product is in a partially or totally molten state.

2.  The process according to claim 1, wherein the foaming step is performed at a temperature above the crystallization temperature (Tc) of the target polymer, preferably at or above the melting temperature (Tm) of said polymer.

3.  The process according to claim 1 or 2, wherein the step of foaming is implemented with a physical foaming agent, preferably selected from gas, more preferably selected from the group consisting in nitrogen, carbon dioxide, methane, helium, neon, argon, xenon, hydrogen or a mixture thereof.

4.  The process according to any one of the previous claims, wherein the step of foaming is implemented with a chemical foaming agent, preferably selected from the group consisting in citric acid, carbonate, bicarbonate or mixture thereof, more preferably a mix of citric acid and bicarbonate.

5.  The process according to any one of the previous claims, further comprising a step of cooling the at least partially foamed plastic product, less than 30 seconds after the foaming step, by submitting the plastic product to a temperature below the crystallization temperature (Tc) of the target polymer, preferably below the glass transition temperature (Tg) of said polymer.

6.  The process according to claim 5, wherein the at least partially foamed plastic product is submitted to a granulation step between the cooling step and the depolymerization step.

7.  The process according to any one of the previous claims, wherein the step of foaming is performed in an extruder or in an autoclave.

8.  The process according to any one of the previous claims, wherein the depolymerizing step comprises contacting the plastic product with a depolymerizing agent selected from chemical and/or biological depolymerizing agents, preferably with a biological depolymerizing agent which is a depolymerase, more preferably with a biological depolymerizing agent which is a depolymerase able to degrade at least one polymer of the plastic product, even more preferably with a biological depolymerizing agent which is a depolymerase able to degrade at least the target polymer of the plastic product.

9.  The process according to any one of the previous claims, wherein the plastic product comprises at least one thermoplastic polymer selected from polyester and/or polyamide and/or polyolefin, and wherein the polyester is preferably selected from polyethylene terephthalate (PET), polytrimethylene terephthalate (PTT), polybutylen terephthalate (PBT), polyethylene isosorbide terephthalate (PEIT), polylactic acid (PLA), polyhydroxy alkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA), polybutylene succinate terephthalate (PBST), polyethylene succinate (PES), poly(butylene succinate/terephthalate/isophthalate)-co-(lactate) (PBSTIL) and blends/mixtures of these materials, even more preferably selected from polyethylene terephthalate and polylactic acid, the polyamide is preferably selected from polyamide-6 or poly($\beta$-caprolactam) or polycaproamide (PA6), polyamide-6,6 or poly(hexamethylene adipamide) (PA6,6), poly(11-aminoundecanoamide) (PA11), polydodecanolactam (PA12), poly(tetramethylene adipamide) (PA4,6), poly(pentamethylene sebacamide) (PA5,10), poly(hexamethylene azelaamide) (PA6,9), poly(hexamethylene sebacamide) (PA6,10), poly(hexamethylene dodecanoamide) (PA6,12), poly(m-xylylene adipamide) (PAMXD6), polyhexamethylene adipamide/polyhexamethyleneterephthalamide copolymer (PA66/6T), polyhexamethylene adipamide/polyhexamethyleneisophthalamide copolymer (PA66/6I) and blends/mixtures of these materials, and the polyolefin is preferably selected from polyethylene, polypropylene, polymethylpentene, polybutene-1, polyisobutylene, ethylene propylene rubber, ethylene propylene diene monomer rubber, ethylene vinyl alcohol, ethylene-carbon monoxide copolymer and copolymers and modifications thereof.

10. A process for degrading a plastic product comprising at least PET comprising the steps of:

> a. Foaming at least partially the plastic product with a chemical foaming agent, wherein the foaming step is performed at a temperature above 170°C, preferably at or above 230°C, wherein the foaming agent is preferably selected from citrate, carbonate, bicarbonate and a mixture thereof;
> b. Cooling said at least partially foamed plastic product at a temperature below 100°C, preferably below 90°C, preferably less than 30 seconds after the foaming step;
> c. Depolymerizing PET of the cooled plastic product; and optionally recovering and optionally purifying oligomers and/or monomers resulting from depolymerization of said PET,

> wherein the step (c) of depolymerization is preferably performed by contacting the cooled plastic product with a biological depolymerizing agent, preferably a depolymerase, more preferably an esterase, even more preferably a cutinase or a lipase.

11. A method of producing monomers and/or oligomers and/or degradation products from a plastic product comprising at least one polymer, preferably PET, comprising submitting successively the plastic product to a foaming step, and to a depolymerizing step, preferably by exposing the foamed plastic product to a depolymerase, preferably an esterase, more preferably a cutinase.

12. A process for degrading a at least partially foamed plastic product comprising at least one polymer, wherein the at least partially foamed plastic product is contacted with a depolymerizing agent able to degrade at least one polymer of said plastic product, and wherein said at least partially foamed plastic product is obtained from plastic waste and/or fiber waste that has been previously submitted to a foaming step.

13. The process of claim 12, wherein said polymer of said at least partially foamed plastic product has been previously amorphized before being in contacted with the depolymerizing agent.

14. A process for recycling a plastic product selected from plastic waste and/or fiber waste comprising at least one polymer, said process comprising the step of depolymerizing at least one target polymer of said plastic product, wherein the plastic product has been previously at least partially foamed.

15. The process of claim 14, wherein the polymer of said plastic product has been previously amorphized.

16. The process of any one of claims 12 to 15, wherein the depolymerization step is performed by submitting the at least partially foamed plastic product to a biological depolymerizing agent, preferably a depolymerase, more preferably a depolymerase able to degrade at least one polymer of the plastic product.

**Patentansprüche**

1. Verfahren zum Abbau eines Kunststoffprodukts, das mindestens ein Polymer enthält, umfassend die folgenden Schritte:

    a. Zumindest teilweises Aufschäumen des Kunststoffprodukts; und
    b. Depolymerisieren mindestens eines Zielpolymers des zumindest teilweise aufgeschäumten Kunststoffprodukts,

wobei der Aufschäumschritt bei einer Temperatur durchgeführt wird, bei der das Kunststoffprodukt teilweise oder vollständig geschmolzen ist.

2. Das Verfahren nach Anspruch 1, wobei der Aufschäumschritt bei einer Temperatur oberhalb der Kristallisationstemperatur (Tc) des Zielpolymers, vorzugsweise bei oder oberhalb der Schmelztemperatur (Tm) dieses Polymers, durchgeführt wird.

3. Das Verfahren nach Anspruch 1 oder 2, wobei der Aufschäumschritt mit einem physikalischen Aufschäummittel durchgeführt wird, vorzugsweise ausgewählt aus Gasen, insbesondere ausgewählt aus der Gruppe bestehend aus Stickstoff, Kohlendioxid, Methan, Helium, Neon, Argon, Xenon, Wasserstoff oder einer Mischung davon.

4. Das Verfahren nach einem der vorhergehenden Ansprüche, wobei der Aufschäumschritt mit einem chemischen Aufschäummittel durchgeführt wird, vorzugsweise ausgewählt aus der Gruppe bestehend aus Zitronensäure, Carbonat, Bicarbonat oder einer Mischung davon, besonders bevorzugt einer Mischung aus Zitronensäure und Bicarbonat.

5. Das Verfahren nach einem der vorhergehenden Ansprüche, umfassend einen Schritt des Abkühlens des zumindest teilweise aufgeschäumten Kunststoffprodukts weniger als 30 Sekunden nach dem Aufschäumschritt, indem das Kunststoffprodukt einer Temperatur unterhalb der Kristallisationstemperatur (Tc) des Zielpolymers, vorzugsweise unterhalb der Glasübergangstemperatur (Tg) dieses Polymers, ausgesetzt wird.

6. Das Verfahren nach Anspruch 5, wobei das zumindest teilweise aufgeschäumte Kunststoffprodukt zwischen dem Abkühlschritt und dem Depolymerisationsschritt einem Granulierungsschritt unterzogen wird.

7. Das Verfahren nach einem der vorhergehenden Ansprüche, wobei der Aufschäumschritt in einem Extruder oder einem Autoklaven durchgeführt wird.

8. Das Verfahren nach einem der vorhergehenden Ansprüche, wobei der Depolymerisationsschritt das Inkontaktbringen des Kunststoffprodukts mit einem Depolymerisationsmittel umfasst, das aus chemischen und/oder biologischen Depolymerisationsmitteln ausgewählt ist, vorzugsweise mit einem biologischen Depolymerisationsmittel, bei dem es sich um eine Depolymerase handelt, stärker bevorzugt mit einem biologischen Depolymerisationsmittel, bei dem es sich um eine Depolymerase handelt, die in der Lage ist, mindestens ein Polymer des Kunststoffprodukts abzubauen, noch stärker bevorzugt mit einem biologischen Depolymerisationsmittel, bei dem es sich um eine Depolymerase handelt, die in der Lage ist, mindestens das Zielpolymer des Kunststoffprodukts abzubauen.

9. Das Verfahren nach einem der vorhergehenden Ansprüche, wobei das Kunststoffprodukt mindestens ein thermoplastisches Polymer umfasst, ausgewählt aus Polyester und/oder Polyamid und/oder Polyolefin, und wobei der Polyester vorzugsweise ausgewählt ist aus Polyethylenterephthalat (PET), Polytrimethylenterephthalat (PTT), Polybutylenterephthalat (PBT), Polyethylenisosorbidterephthalat (PEIT), Polymilchsäure (PLA), Polyhydroxyalkanoat (PHA), Polybutylensuccinat (PBS), Polybutylensuccinatadipat (PBSA), Polybutylenadipatterephthalat (PBAT), Polyethylenfuranoat (PEF), Polycaprolacton (PCL), Poly(ethylenadipat) (PEA), Polybutylensuccinatterephthalat (PBST), Polyethylensuccinat (PES), Poly(butylensuccinat/terephthalat/isophthalat)-co-(lactat) (PBSTIL) und Blends/Mischungen dieser Materialien, noch bevorzugter ausgewählt aus Polyethylenterephthalat und Polymilchsäure, das Polyamid vorzugsweise ausgewählt ist aus Polyamid-6 oder Poly(β-caprolactam) oder Polycaproamid (PA6), Polyamid-6,6 oder **Poly(hexamethylenadipamid)** (PA6,6), Poly(11-aminoundecanoamid) **(PA11),** Polydodecanolactam (PA12), Poly(tetramethylenadipamid) (PA4,6), Poly(pentamethylensebacamid) (PA5,10), Poly(hexamethylenazelaamid) (PA6,9), Poly(hexamethylensebacamid) (PA6,10), Poly(hexamethylendodecanoamid) (PA6,12), Poly(m-xylylenadipamid) (PAMXD6), Polyhexamethylenadipamid/Polyhexamethylenterephthalamid-Copolymer (PA66/6T), Polyhexamethylenadipamid/Polyhexamethylenisophthalamid-Copolymer (PA66/6I) und Blends/Mischungen dieser Materialien und das Polyolefin vorzugsweise ausgewählt ist aus Polyethylen, Polypropylen, Polymethylpenten, Polybuten-1, Polyisobutylen, Ethylen-Propylen-Kautschuk, Ethylen-Propylen-Dien-Monomer-Kautschuk, Ethylen-Vinylalkohol, Ethylen-Kohlenmonoxid-Copolymer und Copolymeren und Modifikationen davon.

10. Verfahren zum Abbau eines Kunststoffprodukts, das mindestens PET enthält, umfassend die folgenden Schritte:

    a. Zumindest teilweises Aufschäumen des Kunststoffprodukts mit einem chemischen Auschäummittel, wobei der Aufschäumschritt bei einer Temperatur über 170 °C, vorzugsweise bei oder über 230 °C, durchgeführt wird, wobei das Aufschäummittel vorzugsweise ausgewählt wird aus Citrat, Carbonat, Bicarbonat und einer Mischung davon;
    b. Abkühlen dieses zumindest teilweise aufgeschäumten Kunststoffprodukts auf eine Temperatur unter 100 °C, vorzugsweise unter 90 °C, vorzugsweise weniger als 30 Sekunden nach dem Aufschäumschritt;
    c. Depolymerisieren des PET des abgekühlten Kunststoffprodukts; und gegebenenfalls Rückgewinnen und gegebenenfalls Reinigen der aus der Depolymerisation dieses PET resultierenden Oligomere und/oder Monomere,

    wobei der Depolymerisationschritt (c) vorzugsweise durch Inkontaktbringen des abgekühlten Kunststoffprodukts mit einem biologischen Depolymerisationsmittel, vorzugsweise einer Depolymerase, bevorzugter einer Esterase, noch bevorzugter einer Cutinase oder einer Lipase, durchgeführt wird.

11. Verfahren zur Herstellung von Monomeren und/oder Oligomeren und/oder Abbauprodukten aus einem Kunststoffprodukt, das mindestens ein Polymer, vorzugsweise PET, umfasst, wobei das Kunststoffprodukt nacheinander, einem Aufschäumschritt und einem Depolymerisationsschritt, vorzugsweise indem das aufgeschäumte Kunststoffprodukt einer Depolymerase, vorzugsweise einer Esterase, stärker bevorzugt einer Cutinase, ausgesetzt wird, unterzogen wird.

12. Verfahren zum Abbau eines zumindest teilweise aufgeschäumten Kunststoffprodukts, das mindestens ein Polymer enthält, wobei das zumindest teilweise aufgeschäumte Kunststoffprodukt mit einem Depolymerisationsmittel in Kontakt gebracht wird, das mindestens ein Polymer dieses Kunststoffprodukts abbauen kann, und wobei dieses zumindest teilweise aufgeschäumte Kunststoffprodukt aus Kunststoffabfällen und/oder Faserabfällen gewonnen wird, die zuvor einem Aufschäumungsschritt unterzogen wurden.

13. Das Verfahren nach Anspruch 12, wobei dieses Polymer dieses zumindest teilweise aufgeschäumten Kunststoffprodukts vor dem Kontakt mit dem Depolymerisationsmittel amorphisiert wurde.

14. Verfahren zum Recycling eines Kunststoffprodukts, ausgewählt aus Kunststoffabfällen und/oder Faserabfällen, das mindestens ein Polymer enthält, wobei dieses Verfahren den Schritt der Depolymerisation mindestens eines Zielpolymers des Kunststoffprodukts umfasst, wobei das Kunststoffprodukt zuvor zumindest teilweise aufgeschäumt wurde.

15. Das Verfahren nach Anspruch 14, wobei das Polymer dieses Kunststoffprodukts zuvor amorphisiert wurde.

16. Das Verfahren nach einem der Ansprüche 12 bis 15, wobei der Depolymerisationsschritt durchgeführt wird, indem das zumindest teilweise aufgeschäumte Kunststoffprodukt einem biologischen Depolymerisationsmittel ausgesetzt wird, vorzugsweise einer Depolymerase, noch bevorzugter einer Depolymerase, die in der Lage ist, mindestens ein Polymer des Kunststoffprodukts abzubauen.

**Revendications**

1. Procédé pour dégrader un produit plastique comprenant au moins un polymère, ledit procédé comprenant les étapes de :

    a. expansion au moins partielle du produit plastique ; et
    b. dépolymérisation d'au moins un polymère visé du produit plastique au moins partiellement expansé,

    dans lequel l'étape d'expansion est effectuée à une température à laquelle le produit plastique est dans un état partiellement ou totalement fondu.

2. Procédé selon la revendication 1, dans lequel l'étape d'expansion est effectuée à une température supérieure à la température de de cristallisation (Tc) du polymère visé, de préférence à une température égale ou supérieure à la température de fusion (Tm) dudit polymère.

3. Procédé selon la revendication 1 ou 2, dans lequel l'étape d'expansion est mise en œuvre avec un agent d'expansion physique, de préférence choisi parmi les gaz, mieux encore choisi dans le groupe constitué par l'azote, le dioxyde de carbone, le méthane, l'hélium, le néon, l'argon, le xénon, l'hydrogène, et leurs mélanges.

4. Procédé selon l'une quelconque des revendications précédentes, dans lequel l'étape d'expansion est mise en œuvre avec un agent d'expansion chimique, de préférence choisi dans le groupe constitué par l'acide citrique, un carbonate, un bicarbonate, et leurs mélanges, mieux encore un mélange d'acide citrique et de bicarbonate.

5. Procédé selon l'une quelconque des revendications précédentes, comprenant en outre une étape de refroidissement du produit plastique au moins partiellement expansé, moins de 30 secondes après l'étape d'expansion, par soumission du produit plastique à une température inférieure à la température de cristallisation (Tc) du polymère visé, de préférence inférieure à la température de transition vitreuse (Tg) dudit polymère.

6. Procédé selon la revendication 5, dans lequel le produit plastique au moins partiellement expansé est soumis à une étape de granulation entre l'étape de refroidissement et l'étape de dépolymérisation.

7. Procédé selon l'une quelconque des revendications précédentes, dans lequel l'étape d'expansion est effectuée dans une extrudeuse ou dans un autoclave.

8. Procédé selon l'une quelconque des revendications précédentes, dans lequel l'étape de dépolymérisation comprend la mise en contact du produit plastique avec un agent de dépolymérisation choisi parmi les agents de dépolymérisation chimiques et/ou biologiques, de préférence avec un agent de dépolymérisation biologique qui est une dépolymérase, mieux encore avec un agent de dépolymérisation biologique qui est une dépolymérase capable de dégrader au moins un polymère du produit plastique, plus particulièrement avec un agent de dépolymérisation biologique qui est une dépolymérase capable de dégrader au moins le polymère visé du produit plastique.

9. Procédé selon l'une quelconque des revendications précédentes, dans lequel le produit plastique comprend au moins un polymère thermoplastique choisi parmi les polyesters et/ou les polyamides et/ou les polyoléfines, et dans lequel le polyester est de préférence choisi parmi le poly(téréphtalate d'éthylène) (PET), le poly(téréphtalate de triméthylène) (PTT), le poly(téréphtalate de butylène) (PBT), le poly(téréphtalate d'éthylène-isosorbide) (PEIT), le poly(acide lactique) (PLA), un polyhydroxyalcanoate (PHA), le poly(succinate de butylène) (PBS), le poly(succinate-adipate de butylène) (PBSA), le poly(adipate-téréphtalate de butylène) (PBAT), le poly(furanoate d'éthylène) (PEF), la polycaprolactone (PCL), le poly(adipate d'éthylène) (PEA), le poly(succinate-téréphtalate de butylène) (PBST), le poly(succinate d'éthylène) (PES), le poly(succinate/ téréphtalate/isophtalate-co-lactate de butylène) (PBSTIL) et les combinaisons/mélanges de ces matériaux, mieux encore choisi parmi le poly(téréphtalate d'éthylène) et le poly(acide lactique), le polyamide est de préférence choisi parmi le polyamide-6 et le poly(β-caprolactame) et le polycaproamide (PA6), le polyamide-6,6 et le poly(hexaméthylène-adipamide) (PA6,6), le poly(11-aminoundécanoamide) (PA11), le polydodécanolactame (PA12), le poly(tétraméthylène-adipamide) (PA4,6), le poly(pentaméthylène-sébaçamide) (PA5,10), le poly(hexaméthylène-azélaamide) (PA6,9), le poly(hexaméthylène-sébaçamide) (PA6,10), le poly(hexaméthylène-dodécanoamide) (PA6,12), le poly(m-xylylène-apidamide) (PAMXD6), le copolymère de poly(hexaméthylène-adipamide)/poly(hexaméthylène-téréphtalamide) (PA66/6T), le copolymère de poly(hexaméthylène-adipamide)/poly(hexaméthylène-isophtalamide) (PA66/6I) et les combinaisons/mélanges de ces matériaux, et la polyoléfine est de préférence choisie parmi le polyéthylène, le polypropylène, le polyméthylpentène, le polybutène-1, le polyisobutylène, le caoutchouc d'éthylène-propylène, le caoutchouc de monomère éthylène-propylène-diène, l'éthylène-alcool vinylique, le copolymère d'éthylène-monoxyde de carbone, et leurs copolymères et modifications.

10. Procédé pour dégrader un produit plastique comprenant au moins du PET, comprenant les étapes de :

   a. expansion au moins partielle du produit plastique avec un agent d'expansion chimique, laquelle étape d'expansion est effectuée à une température supérieure à 170 °C, de préférence égale ou supérieure à 230 °C, dans laquelle l'agent d'expansion est de préférence choisi parmi un citrate, un carbonate, un bicarbonate et un mélange de ceux-ci ;

b. refroidissement dudit produit plastique au moins partiellement expansé à une température inférieure à 100 °C, de préférence inférieure à 90 °C, de préférence moins de 30 secondes après l'étape d'expansion ;
c. dépolymérisation du PET du produit plastique refroidi ; et éventuellement récupération et éventuellement purification d'oligomères et/ou de monomères résultant de la dépolymérisation dudit PET,

dans lequel l'étape (c) de dépolymérisation est de préférence effectuée par mise en contact du produit plastique refroidi avec un agent de dépolymérisation biologique, de préférence une dépolymérase, mieux encore une estérase, plus particulièrement une cutinase ou une lipase.

11. Méthode de production de monomères et/ou d'oligomères et/ou de produits de dégradation à partir d'un produit plastique comprenant au moins un polymère, de préférence du PET, comprenant la soumission du produit plastique successivement à une étape d'expansion et à une étape de dépolymérisation, de préférence par exposition du produit plastique expansé à une dépolymérase, de préférence à une estérase, mieux encore à une cutinase.

12. Procédé pour dégrader un produit plastique au moins partiellement expansé comprenant au moins un polymère, dans lequel le produit plastique au moins partiellement expansé est mis en contact avec un agent de dépolymérisation capable de dégrader au moins un polymère dudit produit plastique, et dans lequel ledit produit plastique au moins partiellement expansé est obtenu à partir de déchets plastiques et/ou de déchets fibreux qui ont été préalablement soumis à une étape d'expansion.

13. Procédé selon la revendication 12, dans lequel ledit polymère dudit produit plastique au moins partiellement expansé a été préalablement amorphisé avant sa mise en contact avec l'agent de dépolymérisation.

14. Procédé pour recycler un produit plastique choisi parmi les déchets plastiques et/ou les déchets fibreux comprenant au moins un polymère, ledit procédé comprenant l'étape de dépolymérisation d'au moins un polymère visé dudit produit plastique, dans lequel le produit plastique a été préalablement au moins partiellement expansé.

15. Procédé selon la revendication 14, dans lequel le polymère dudit produit plastique a été préalablement amorphisé.

16. Procédé selon l'une quelconque des revendications 12 à 15, dans lequel l'étape de dépolymérisation est effectuée par soumission du produit plastique au moins partiellement expansé à un agent de dépolymérisation biologique, de préférence à une dépolymérase, mieux encore à une dépolymérase capable de dégrader au moins un polymère du produit plastique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014079844 A
- WO 2015097104 A
- WO 2015173265 A
- WO 2017198786 A
- EP 1528079 A1
- US 6255451 B1
- US 5212223 A

- CN 102532647 A
- EP 3517608 A, Sulaiman
- WO 2018011284 A
- WO 2018011281 A
- WO 2016062695 A
- WO 2018109183 A
- WO 2019122308 A

**Non-patent literature cited in the description**

- Polymer Data Handbook. OXFORD, 2009
- **FISHER E. W.** ; **STERZEL H. J.** ; **WEGNER G.** Investigation of the structure of solution grown crystals of lactide copolymers by means of chemical reactions. *Kolloid Zeitschrift & Zeitschrift fur Polymere*, 1973, vol. 251, 980-990

- **SULAIMAN et al.** *Appl Environ Microbiol.*, March 2012
- *Journal of Physical and Chemical Reference Data*, 1996, vol. 25 (6), 1509-1596